(19) Europäisches Patentamt
European Patent Office
Office européen des brevets

(11) **EP 4 653 673 A1**

(12) **EUROPÄISCHE PATENTANMELDUNG**

(43) Veröffentlichungstag:
   **26.11.2025  Patentblatt 2025/48**

(21) Anmeldenummer: **25177199.4**

(22) Anmeldetag: **19.05.2025**

(51) Internationale Patentklassifikation (IPC):
   **F01N 11/00** (2006.01)      **G01N 33/00** (2006.01)

(52) Gemeinsame Patentklassifikation (CPC):
   **F01N 11/00; A01K 29/00; G01N 33/0054;**
   F01N 2560/02; F01N 2560/021; F01N 2560/026

(84) Benannte Vertragsstaaten:
   **AL AT BE BG CH CY CZ DE DK EE ES FI FR GB
   GR HR HU IE IS IT LI LT LU LV MC ME MK MT NL
   NO PL PT RO RS SE SI SK SM TR**
   Benannte Erstreckungsstaaten:
   **BA**
   Benannte Validierungsstaaten:
   **GE KH LA MA MD TN**

(30) Priorität:  **24.05.2024  DE 102024114599
   07.08.2024  DE 102024122491**

(71) Anmelder: **Dräger Safety AG & Co. KGaA
   23560 Lübeck (DE)**

(72) Erfinder: **Wruck, Norbert
   23558 Lübeck (DE)**

(54) **ANORDNUNG UND VERFAHREN ZUM ÜBERWACHEN EINER ABGASREINIGUNGSANLAGE FÜR AMMONIAK**

(57)    Die Erfindung betrifft eine Anordnung und ein Verfahren, welche eine Abgasreinigungsanlage (ARA) überwachen. Die Abgasreinigungsanlage (ARA) reduziert den Gehalt von Ammoniak in einem Rohgas und liefert dadurch ein Reingas. Ein Rohgas-Sensor (1.i1, 1.i2) misst den Ammoniak-Gehalt im Rohgas, also flussaufwärts von der Abgasreinigungsanlage (ARA). Ein Reingas-Sensor (1.o1, 1.o2) misst den Ammoniak-Ge-halt im Reingas, also flussabwärts von der Abgasreinigungsanlage (ARA). Eine signalverarbeitende Auswertungseinheit (8) ermittelt jeden Fehler-Zeitraum. In einem Fehler-Zeitraum ist eine vorgegebene Gütefunktion kleiner als eine vorgegebene untere Schranke. Die Gütefunktion ist umso größer, je kleiner bei gleichbleibendem Ammoniak-Gehalt im Rohgas der Ammoniak-Gehalt im Reingas ist.

FIG. 1

EP 4 653 673 A1

**Beschreibung**

Die Erfindung betrifft eine Anordnung und ein Verfahren, welche mindestens eine Abgasreinigungsanlage zu überwachen vermögen. Die oder jede überwachte Abgasreinigungsanlage ist dazu ausgestaltet, den Gehalt von Ammoniak ($NH_3$) in einem Gasgemisch zu reduzieren.

Der Begriff "Abgas" bezeichnet allgemein ein Gasgemisch, welches in einem Herstellungs- oder Verarbeitungsprozess entsteht, wobei das Gasgemisch mindestens einen Schadstoff enthält. Ein Schadstoff ist eine für Menschen und / oder für die Umwelt schädliche Substanz, und daher soll der Gehalt (die Konzentration) des Schadstoffs in einem für Menschen zugänglichen Bereich, insbesondere in der Umgebung, unter einer vorgegebenen oberen Schranke liegen. Falls das Gasgemisch im Wesentlichen aus Atemluft besteht, wird auch die Bezeichnung "Abluft" als Sonderfall für Abgas verwendet.

Eine derartige Abgasreinigungsanlage wird beispielsweise in einem landwirtschaftlichen Betrieb zur Tierzucht und in Anlagen zur Aufbereitung von Klärschlamm oder in einer Anlage zur Erzeugung von chemischen Stoffen oder von Nahrungsmitteln eingesetzt. In Anlagen wie den gerade genannten entsteht häufig Ammoniak. Die Abgasreinigungsanlage wird dafür verwendet, um die Menge von Ammoniak, das in die Umgebung austritt, zu reduzieren und idealerweise vollständig zu verhindern, dass Ammoniak in die Umgebung austritt.

Der Erfindung liegt die Aufgabe zugrunde, eine Überwachungs-Anordnung und ein Überwachungs-Verfahren bereitzustellen, welche eine Abgasreinigungsanlage für Ammoniak zu überwachen vermögen und die Notwendigkeit ersparen, dass dauerhaft ein Mensch die Anlage überwachen muss.

Die Aufgabe wird durch eine Überwachungs-Anordnung mit den Merkmalen des Anspruchs 1 und durch ein Überwachungs-Verfahren mit den Merkmalen des Anspruchs 18 gelöst. Vorteilhafte Ausgestaltungen sind in den Unteransprüchen angegeben. Vorteilhafte Ausgestaltungen der erfindungsgemäßen Überwachungs-Anordnung sind, soweit sinnvoll, auch vorteilhafte Ausgestaltungen des erfindungsgemäßen Überwachungs-Verfahrens und umgekehrt.

Die erfindungsgemäße Überwachungs-Anordnung und das erfindungsgemäße Überwachungs-Verfahren vermögen mindestens eine Abgasreinigungsanlage zu überwachen, optional gleichzeitig oder wenigstens zeitlich überlappend mehrere Abgasreinigungsanlagen. Mehrere überwachte Abgasreinigungsanlagen können an mindestens zwei unterschiedlichen Orten aufgestellt sein. Jeweils ein Gasgemisch fließt zu der oder jeder überwachten Abgasreinigungsanlage. Dieses Gasgemisch enthält Ammoniak oder kann wenigstens zeitweise Ammoniak aufweisen. Das Gasgemisch, welches das Ammoniak enthält oder enthalten kann und welches die überwachte Abgasreinigungsanlage erreicht, wird nachfolgend "Rohgas" genannt. Die oder jede überwachte Abgasreinigungsanlage vermag den Gehalt von Ammoniak in diesem Gasgemisch zu senken - natürlich nur dann, wenn das Rohgas Ammoniak enthält. Das Gasgemisch, in welchem der Ammoniak-Gehalt im Vergleich zum Rohgas reduziert ist und welches die Abgasreinigungsanlage verlässt, wird nachfolgend "Reingas" genannt. Idealerweise enthält das Reingas überhaupt kein Ammoniak mehr.

Die Überwachungs-Anordnung umfasst eine erste Überwachungseinheit. Falls die Überwachungs-Anordnung mehrere Abgasreinigungsanlagen gleichzeitig oder wenigstens zeitlich überlappend überwacht, so umfasst die Überwachungs-Anordnung für jede einzelne überwachte Abgasreinigungsanlage jeweils eine Überwachungseinheit. Die oder jede Überwachungseinheit ist also jeweils einer überwachten Abgasreinigungsanlage zugeordnet. Das Überwachungs-Verfahren wird unter Verwendung einer derartigen Überwachungs-Anordnung durchgeführt.

Die erste Überwachungseinheit umfasst

- mindestens einen Rohgas-Sensor, optional mehrere Rohgas-Sensoren, und
- mindestens einen Reingas-Sensor, optional mehrere Reingas-Sensoren.

Die oder jede optionale weitere Überwachungseinheit umfasst ebenfalls jeweils mindestens einen Rohgas-Sensor und mindestens einen Reingas-Sensor.

Der oder jeder Rohgas-Sensor ist dazu ausgestaltet, den Ammoniak-Gehalt (die Ammoniak-Konzentration, den Ammoniak-Anteil) im Rohgas zu messen. Dies bedeutet: Der Rohgas-Sensor vermag mindestens eine physikalische Größe zu messen, die mit dem Ammoniak-Gehalt im Rohgas korreliert. Dieses Rohgas erreicht die überwachte Abgasreinigungsanlage. Die gemessene Größe oder die Kombination der gemessenen Größen zusammen korrelieren mit dem Ammoniak-Gehalt im Rohgas und sind daher ein Maß für den Ammoniak-Gehalt im Rohgas.

Anmerkung: Die Begriffe "Rohgas-Sensor" und "Reingas-Sensor" spezifizieren die Verwendung eines Ammoniak-Sensors. Ein Rohgas-Sensor kann genauso aufgebaut sein wie ein Reingas-Sensor.

Der oder jeder Rohgas-Sensor vermag jeweils ein Signal zu generieren, wobei dieses Signal eine Information über den von diesem Sensor gemessenen Ammoniak-Gehalt im Rohgas enthält.

Der oder jeder Reingas-Sensor ist dazu ausgestaltet, den Ammoniak-Gehalt im Reingas zu messen. Dieses Reingas verlässt die überwachte Abgasreinigungsanlage. Dies bedeutet: Der Reingas-Sensor vermag mindestens eine physikalische Größe zu messen, die mit dem Ammoniak-Gehalt im Reingas korreliert.

Der oder jeder Reingas-Sensor vermag jeweils ein Signal zu generieren, wobei dieses Signal eine Information über den von diesem Sensor gemessenen Ammoniak-Gehalt im Reingas enthält.

Falls nicht anders spezifiziert, bezeichnet der nachfolgend verwendete Begriff "Sensor" sowohl den oder jeden Rohgas-Sensor als auch den oder jeden Reingas-Sensor der ersten Überwachungseinheit. Nachfolgend wird die Situation beschrieben, dass die Überwachungs-Anordnung eine Abgasreinigungsanlage überwacht und eine zugeordnete erste Überwachungseinheit umfasst. Eine entsprechende Abwandlung gilt für die Ausgestaltung, dass die Überwachungs-Anordnung mindestens zwei Abgasreinigungsanlagen überwacht und für jede überwachte Abgasreinigungsanlage jeweils eine zugeordnete Überwachungseinheit umfasst.

Die Überwachungs-Anordnung umfasst weiterhin eine signalverarbeitende Auswertungseinheit. Die Auswertungseinheit kann als ein Softwareprogramm realisiert sein oder ein Softwareprogramm umfassen. Ein Prozessor eines Rechners vermag die Auswertungseinheit auszuführen. Bei der Ausführung bewirkt die Auswertungseinheit die nachfolgend beschriebenen Schritte. Die Auswertungseinheit kann auch als ein Prozessor oder als eine Signalverarbeitungseinheit realisiert sein oder einen Prozessor umfassen, wobei der Prozessor dazu ausgestaltet ist, die nachfolgend beschriebenen Schritte durchzuführen.

Die Auswertungseinheit vermag den Ammoniak-Gehalt im Rohgas und den Ammoniak-Gehalt im Reingas der überwachten Abgasreinigungsanlage zu ermitteln. Für diese Ermittlung vermag die Auswertungseinheit Nachrichten zu empfangen und zu verarbeiten. Die empfangenen und verarbeiteten Nachrichten umfassen die Signale der Sensoren der ersten Überwachungseinheit.

Die Auswertungseinheit vermag jeden Fehler-Zeitraum, der in einem vorgegebenen Überwachungs-Zeitraum auftritt, zu ermitteln. Für diese Ermittlung vermag die Auswertungseinheit den ermittelten (gemessenen) Ammoniak-Gehalt im Rohgas und den ermittelten Ammoniak-Gehalt im Reingas zu verwenden. Alternativ vermag die Auswertungseinheit festzustellen, dass es im Überwachungs-Zeitraum keinen Fehler-Zeitraum gibt.

In einer rechnerausführbaren Form ist eine Gütefunktion vorgegeben. Diese Gütefunktion hängt vom Ammoniak-Gehalt im Reingas und vom Ammoniak-Gehalt im Rohgas ab. Die Gütefunktion hat folgende Eigenschaft: Der Funktionswert der Gütefunktion ist umso größer, je kleiner bei gleichbleibendem Ammoniak-Gehalt im Rohgas der Ammoniak-Gehalt im Reingas ist. Der Funktionswert der Gütefunktion ist also umso größer, je mehr Ammoniak die Abgasreinigungsanlage aus dem Rohgas entfernt.

Ein Fehler-Zeitraum ist ein Zeitraum, in dem jeder ermittelte Funktionswert der Gütefunktion kleiner als eine vorgegebene untere Schranke ist.

In einer Ausgestaltung ist ein Funktionswert der Gütefunktion umso größer, je kleiner der Quotient aus dem Ammoniak-Gehalt im Reingas (Zähler) und dem Ammoniak-Gehalt im Rohgas (Nenner) ist. In einer anderen Ausgestaltung ist der Funktionswert der Gütefunktion umso größer, je größer der Quotient aus $\Delta$ (Zähler) und dem Ammoniak-Gehalt im Rohgas (Nenner) ist, wobei $\Delta$ die Differenz aus dem Ammoniak-Gehalt im Rohgas und dem Ammoniak-Gehalt im Reingas ist. Wenn die Abgasreinigungsanlage nicht vollständig ausgefallen ist und das Rohgas Ammoniak enthält, sind $\Delta > 0$ und der Nenner $> 0$. Diese Gütefunktion lässt sich auch als relativer Reinigungsgrad bezeichnen und ist bestenfalls (höchstens) gleich 1.

Bevorzugt werden bei der Suche nach Fehler-Zeiträumen nur solche Zeiträume aufgefunden, die mindestens so lang sind wie eine vorgegebene Mindestdauer. Dadurch lässt sich in manchen Fällen der Einfluss von Ausreißern reduzieren.

Bevorzugt ist die Überwachungs-Anordnung wie folgt ausgestaltet: Die Auswertungseinheit empfängt von jedem Sensor der ersten Überwachungseinheit jeweils eine Abfolge von Signalwerten, wobei der zeitliche Abstand zwischen zwei aufeinanderfolgenden Signalwerten nicht größer ist als eine vorgegebener zeitlicher Abstand. Ein Zeitraum, der länger ist als dieser vorgegebene zeitliche Abstand und in dem von mindestens einem Sensor kein Signalwert bei der Auswertungseinheit eintrifft, wird ebenfalls als ein Fehler-Zeitraum gewertet. In diesem längeren Zeitraum sind nämlich in der Regel der Sensor und / oder die Datenübertragung vom Sensor oder von der ersten Überwachungseinheit zur Auswertungseinheit ausgefallen.

Das erfindungsgemäße Überwachungs-Verfahren wird automatisch unter Verwendung einer erfindungsgemäßen Überwachungs-Anordnung durchgeführt und umfasst die entsprechenden Schritte.

Gesetzliche und behördliche Vorschriften legen in vielen Fällen fest, dass die Emissionen von Ammoniak in die Umgebung begrenzt sein müssen. Daher ist für viele Betriebe und Anlagen, in denen wenigstens zeitweise Ammoniak entsteht, eine Abgasreinigungsanlage erforderlich. Eine solche Abgasreinigungsanlage entfernt wenigstens einen Teil des Ammoniaks aus einem Gasgemisch, das in dem Betrieb entsteht und als Rohgas fungiert, reduziert also den Ammoniak-Gehalt. In einer Anwendung ist der Betrieb ein landwirtschaftlicher Betrieb, in dem Nutzvieh gehalten und gefüttert wird. In einer anderen Anwendung ist der Betrieb eine Anlage, in der Klärschlamm aufbereitet wird, oder eine Anlage, in der chemische Substanzen oder Nahrungsmittel produziert werden.

Die Erfindung ermöglicht es, die Abgasreinigungsanlage aus der Ferne zu überwachen. Die Sensoren und die Auswertungseinheit arbeiten automatisch, und in der Regel müssen lediglich die Sensoren in der Regel lediglich von Zeit zu Zeit von einem Menschen überprüft werden.

Erfindungsgemäß umfasst die erste Überwachungseinheit mindestens einen Rohgas-Sensor und mindestens einen Reingas-Sensor. Bevorzugt ist jeder Sensor räumlich beabstandet von dem oder jedem anderen Sensor der ersten Überwachungseinheit angeordnet. Der oder jeder Rohgas-Sensor misst den Ammoniak-Gehalt flussaufwärts von der Abgasreinigungsanlage, der oder jeder Reingas-Sensor flussabwärts von derselben Abgasreinigungsanlage. Dank dieses Merkmals ist es nicht erforderlich, den Ammoniak-Gehalt flussabwärts von der Abgasreinigungsanlage aus einem gemessenen Ammoniak-Gehalt flussaufwärts von der Abgasreinigungsanlage herzuleiten oder umgekehrt. Diese Herleitung würde in der Regel erfordern, dass mindestens eine weitere Größe gemessen oder vorgegeben wird, insbesondere eine Größe der überwachten Abgasreinigungsanlage selbst oder eine Umgebungsbedingung. Dank der Erfindung ist es nicht erforderlich, eine solche weitere Größe zu messen oder einen Standardwert vorzugeben. In vielen Fällen vergrößert sich dadurch die Zuverlässigkeit, dass der Ammoniak-Gehalt im Rohgas und der Ammoniak-Gehalt im Reingas jeweils relativ zuverlässig gemessen werden.

Weil ein Rohgas-Sensor und ein Reingas-Sensor verwendet werden, ist es zwar möglich, aber in vielen Fällen nicht erforderlich, einen Betriebsparameter der überwachten Abgasreinigungsanlage zu messen. Beispiele für solche Betriebsparameter sind ein Volumenfluss, den eine Fluidfördereinheit der Abgasreinigungsanlage erzielt, oder ein Stromverbrauch der Fluidfördereinheit oder ein Verbrauch an einer zur Abgasreinigung verwendeten Chemikalien oder ein Volumenfluss oder eine Eigenschaft einer Reinigungsflüssigkeit. Weil es nicht nötig ist, einen Betriebsparameter zu messen, ist es auch nicht erforderlich, die Überwachungs-Anordnung an ein bestimmtes Übertragungsprotokoll oder ein bestimmtes Datenformat der überwachten Abgasreinigungsanlage anzupassen. Außerdem hängt die Zuverlässigkeit, mit der die Überwachungs-Anordnung die Abgasreinigungsanlage überwacht, nicht von einem Sensor der überwachten Abgasreinigungsanlage und von dessen Zuverlässigkeit ab. Das Merkmal, dass kein Parameter der überwachten Abgasreinigungsanlage gemessen zu werden braucht, erleichtert es in vielen Fällen, eine erfindungsgemäße Überwachungs-Anordnung zu realisieren, auch zur Überwachung einer bereits bestehende Abgasreinigungsanlage.

Ein simples Beispiel soll den erfindungsgemäßen Vorteil verdeutlichen. Gemäß diesem Beispiel heizt eine Heizungsanlage ein Gebäude. Festgestellt werden soll, ob das Gebäude ausreichend geheizt wird, also ob die Heizungsanlage korrekt funktioniert. Möglich wäre es, einen Betriebsparameter der Heizungsanlage zu messen, beispielsweise den Verbrauch an fossilem Brennstoff oder elektrische Energie. Dies ist möglich, aber dann nicht notwendig, wenn ein Innen-Thermometer (entspricht dem Reingas-Sensor) die Temperatur im Gebäude und ein Außen-Thermometer (entspricht dem Rohgas-Sensor) die Temperatur außerhalb des Gebäudes misst.

Erfindungsgemäß messen sowohl der oder jeder Rohgas-Sensor als auch der oder jeder Reingas-Sensor jeweils einen Ammoniak-Gehalt (die Ammoniak-Konzentration, den Ammoniak-Anteil) in einem Gasgemisch. Möglich, aber dank der Erfindung in vielen Fällen nicht erforderlich ist es, die Masse von Ammoniak in einem Gasgemisch zu messen. Außerdem ist es möglich, aber dank der Erfindung in vielen Fällen nicht erforderlich, einen Volumenfluss (Volumenstrom) oder einen Massefluss des Ammoniaks oder des gesamten Gasgemischs zu messen, um den Ammoniak-Gehalt herzuleiten. Häufig lässt sich die Konzentration von Ammoniak in einem Gasgemisch mit höherer Zuverlässigkeit messen als die Masse oder der Massenfluss oder der Volumenfluss. Anstelle einer Masse oder eines Massenflusses oder eines Volumenflusses wird in vielen Fällen eine physikalische Größe gemessen, die direkt mit dem Ammoniak-Gehalt in einem Gasgemisch korreliert.

Der ermittelte Ammoniak-Gehalt im Reingas lässt sich dafür verwenden, um die verbleibenden Ammoniak-Emissionen zu ermitteln. Jedoch reicht in vielen Fällen der Ammoniak-Gehalt im Reingas allein nicht aus, um zu entscheiden, ob die Abgasreinigungsanlage noch fehlerfrei arbeitet oder nicht. Falls nämlich das Rohgas wenig Ammoniak enthält, so weist auch das Reingas wenig Ammoniak auf, selbst wenn die Abgasreinigungsanlage fehlerhaft oder gar nicht arbeitet. Ein Extremfall: falls der Ammoniak-Gehalt im Rohgas unter einer vorgegebenen oberen Schranke liegt, so liegt der Ammoniak-Gehalt im Reingas auch unterhalb dieser Schranke, selbst wenn die Abgasreinigungsanlage überhaupt nicht arbeitet. Falls später Rohgas mit einem größeren Volumenstrom oder mit einem größeren Ammoniak-Gehalt der Abgasreinigungsanlage zugeführt wird, so kann eine obere Schranke für die Menge von emittiertem Ammoniak rasch überschritten werden. In vielen Fällen ermöglicht die Erfindung es, eine solche unerwünschte Situation frühzeitig zu erkennen oder sogar gar nicht erst entstehen zu lassen.

Erfindungsgemäß wird der jeweilige Ammoniak-Gehalt sowohl im Reingas als auch im Rohgas, also sowohl flussabwärts als auch flussaufwärts von der Abgasreinigungsanlage, gemessen. Die erfindungsgemäß verwendete Gütefunktion hängt sowohl vom Ammoniak-Gehalt im Reingas als auch vom Ammoniak-Gehalt im Rohgas ab. Die Gütefunktion ist ein Maß dafür, wie gut und zu welchem Grad die Abgasreinigungsanlage den Ammoniak-Gehalt im Rohgas reduziert.

Erfindungsgemäß ermittelt die Auswertungseinheit jeden Fehler-Zeitraum in einem vorgegebenen Überwachungs-Zeitraum. In einem Fehler-Zeitraum nimmt die Gütefunktion durchgehend einen Funktionswert an, der kleiner ist als die vorgegebene untere Schranke. In der Regel schwankt der aktuelle Funktionswert der Gütefunktion sowohl in einem Fehler-Zeitraum als auch außerhalb eines Fehler-Zeitraums. Die ermittelten Fehler-Zeiträume lassen sich in vielen Fällen dafür verwenden, um zu beurteilen, ob in einem Fehler-Zeitraum tatsächlich die Abgasreinigungsanlage fehlerhaft gearbeitet hat oder ob ein anderer Einflussfaktor zu geringen Werten der Gütefunktion geführt hat, beispielsweise wenig Rohgas (insbesondere geringer Volumenfluss oder Massefluss) oder wenig Ammoniak im Rohgas (geringer Ammoniak-Gehalt).

Die erfindungsgemäß verwendete Gütefunktion hängt vom Ammoniak-Gehalt im Rohgas und vom Ammoniak-Gehalt im Reingas ab. Sie hängt nicht direkt von der Menge des Ammoniaks im Rohgas oder von der Menge des Ammoniaks, der als Teil des Reingas emittiert wird, ab. Daher ist die Gütefunktion in vielen Fällen ein besseres Maß für die Güte der Abgasreinigungsanlage als die emittierte Ammoniak-Menge.

Die Erfindung erfordert nicht, ein analytisches Modell oder einen trainierten Klassifikator einzusetzen, wobei das Modell oder der trainierte Klassifikator das Verhalten der Abgasreinigungsanlage beschreibt. Ein solches analytisches Modell aufzustellen und zu überprüfen, erfordert Zeit. In vielen Fällen umfasst ein solches analytische Modell als Modellparameter außerdem Betriebsparameter der überwachten Abgasreinigungsanlage, wobei die Betriebsparameter dann im laufenden Betrieb gemessen werden müssten. Um einen Klassifikator aufzustellen, braucht man eine ausreichend große und verlässliche Stichprobe. Ein weiterer Nachteil eines Klassifikators kann sein, dass eine Stichprobe von einer bestimmten Abgasreinigungsanlage sich nicht für eine andere Abgasreinigungsanlage verwenden lässt.

In einer Ausgestaltung umfasst die erste Überwachungseinheit zwei Rohgas-Sensoren, optional drei oder sogar noch mehr Rohgas-Sensoren. Diese Ausgestaltung ermöglicht es einerseits, den Ammoniak-Gehalt im Rohgas an mindestens zwei verschiedenen Messpositionen zu messen und einen über den Raum gemittelten Ammoniak-Gehalt zu ermitteln. Andererseits ermöglicht diese Ausgestaltung es, den Ausfall eines Rohgas-Sensors zu erkennen und trotz ausgefallenem Rohgas-Sensor die Überwachung der Abgasreinigungsanlage fortzusetzen. Dies wird nachfolgend näher beschrieben.

Die Auswertungseinheit vermag automatisch zu überprüfen, ob ein vorgegebenes Ausfallkriterium für einen der mindestens zwei Rohgas-Sensoren der ersten Überwachungseinheit erfüllt ist oder nicht. Das Ausfallkriterium ist erfüllt, wenn mindestens eine der folgenden Bedingungen eingetreten ist:

- Die absolute oder prozentuale Abweichung zwischen dem Ammoniak-Gehalt, den ein erster Rohgas-Sensor der ersten Überwachungseinheit gemessen hat, und dem Ammoniak-Gehalt, den der oder mindestens ein anderer (zweiter) Rohgas-Sensor der ersten Überwachungseinheit gemessen hat, ist größer als eine vorgegebene untere Schranke. Die gemessenen Werte beziehen sich - bis auf eine Toleranz- auf denselben Zeitpunkt.
- Die Veränderung dieser absoluten oder prozentualen Abweichung ist größer als eine vorgegebene untere Schranke.
- Der zeitliche Verlauf des Ammoniak-Gehalts, den ein Rohgas-Sensor misst, fällt sehr rasch, nämlich rascher als eine vorgegebene obere Schranke, auf einen niedrigen Wert, beispielsweise auf null. Ein sehr rascher Abfall des Ammoniak-Gehalts tritt in der Realität meistens nicht auf.

Falls alle Rohgas-Sensoren der ersten Überwachungseinheit intakt sind, tritt eine solche Situation in der Regel nicht auf. Insbesondere ist das vorgegebene Ausfallkriterium dann erfüllt, wenn ein gemessener Ammoniak-Gehalt plötzlich auf null fällt und bevorzugt ausreichend lange bei null bleibt, ein anderer von derselben Überwachungseinheit gemessener Ammoniak-Gehalt im Rohgas aber nicht.

In aller Regel führt ein Fehler eines Rohgas-Sensors dazu, dass dieser Sensor einen zu geringen Ammoniak-Gehalt im Rohgas misst. Ein Ausfall führt im Extremfall sogar dazu, dass der Sensor überhaupt kein Ammoniak detektiert, obwohl im Rohgas Ammoniak vorhanden ist. In der Regel führt ein Sensor-Fehler aber nicht dazu, dass der fehlerhafte Sensor einen zu hohen Ammoniak-Gehalt misst. Falls der Auswertungseinheit das Ereignis detektiert hat, dass ein Ausfallkriterium erfüllt ist und daher ein Rohgas-Sensor ausgefallen sein muss, so entscheidet die Auswertungseinheit automatisch, welcher Rohgas-Sensor ausgefallen ist und welcher nicht. Diese Entscheidung fällt die Auswertungseinheit wie folgt: Derjenige Sensor wird als ein ausgefallener Sensor behandelt, der den kleineren oder kleinsten oder sehr stark abfallenden Ammoniak-Gehalt im Rohgas misst und ein entsprechendes Signal liefert. Der Messwert dieses Sensors wird nicht verwendet. Mit anderen Worten: Die Auswertungseinheit verwendet den größeren Ammoniak-Gehalt oder allgemeiner bei n Rohgas-Sensoren die n-1 größten gemessenen Ammoniak-Gehalte, um den tatsächlichen Ammoniak-Gehalt zu ermitteln. Falls das Ausfallkriterium nicht erfüllt ist, verwendet die Auswertungseinheit das jeweilige Signal jedes Rohgas-Sensors und aggregiert über die gemessenen Ammoniak-Gehalte, beispielsweise als arithmetisches oder gewichtetes Mittel oder als Median.

Entsprechend umfasst in einer Ausgestaltung die erste Überwachungseinheit mindestens zwei Reingas-Sensoren. Die gerade beschriebene Ausgestaltung, um den Ausfall eines Rohgas-Sensors automatisch zu erkennen, wird bevorzugt entsprechend ebenfalls dafür verwendet, um den Ausfall eines Reingas-Sensors zu erkennen und trotzdem den Ammoniak-Gehalt im Reingas zu messen.

In einer Ausgestaltung vermag die Auswertungseinheit zu ermitteln, welche Verfügbarkeitsrate die Abgasreinigungsanlage in dem vorgegebenen Überwachungs-Zeitraum aufweist. Für diese Ermittlung verwendet die Auswertungseinheit die ermittelten Fehler-Zeiträume und die jeweilige zeitliche Dauer jedes Fehler-Zeitraums, wobei diejenigen Fehler-Zeiträume berücksichtigt werden, die in den Überwachungs-Zeitraum fallen und wobei bevorzugt nur Fehler-Zeiträume berücksichtigt werden, die mindestens so lang wie eine vorgegebene Mindestdauer sind. Erfindungsgemäß nehmen alle Funktionswerte der Gütefunktion in einem Fehler-Zeitraum jeweils einen Wert an, der kleiner als die vorgegebene untere Schranke ist. Außerhalb eines Fehler-Zeitraums nehmen die Funktionswerte der Gütefunktion in der Regel Werte gleich der oder größer als die oben untere Schranke an. Die ermittelte Verfügbarkeitsrate gibt an, welchen zeitlichen Anteil diejenigen Zeiträume, in denen die Gütefunktion größer als oder gleich der unteren Schranke gewesen ist, am Überwachungs-Zeitraum insgesamt hatten. Je kürzer also die Fehler-Zeiträume bei einem vorgegebenen Überwachungs-Zeitraum insgesamt sind, desto größer ist die Verfügbarkeitsrate. Falls die Abgasreinigungsanlage im gesamten Überwachungs-Zeitraum fehlerfrei arbeitet, d.h. kein Fehler-Zeitraum detektiert ist, beträgt die Verfügbarkeitsrate 1. Falls sie im gesamten Überwachungs-Zeitraum fehlerhaft arbeitet, so beträgt die Verfügbarkeitsrate 0.

In einer Ausgestaltung vermag die Auswertungseinheit eine graphische Darstellung zu erzeugen und zu bewirken, dass diese graphische Darstellung visuell ausgegeben wird. Diese graphische Darstellung hat eine erste Achse für die Zeit und eine zweite Achse für den Ammoniak-Gehalt im Reingas. Diesen Ammoniak-Gehalt hat die Auswertungseinheit abhängig von den Signalen der Sensoren der zugeordneten Überwachungseinheit ermittelt. In der Regel ist die erste Achse die x-Achse und die zweite Achse die y-Achse, wobei die y-Achse bevorzugt senkrecht auf der x-Achse steht. Die graphische Darstellung zeigt den zeitlichen Verlauf des ermittelten Ammoniak-Gehalts im Reingas. Außerdem zeigt die graphische Darstellung jeden ermittelten Fehler-Zeitraum. In einer Ausgestaltung zeigt die graphische Darstellung zusätzlich den zeitlichen Verlauf des ermittelten Ammoniak-Gehalts im Rohgas, dies ist aber nicht erforderlich. In der graphischen Darstellung ist jeder Abschnitt des zeitlichen Verlaufs des Ammoniak-Gehalts im Reingas hervorgehoben dargestellt, der in einen Fehler-Zeitraum fällt. Beispielsweise ist die Fläche zwischen dem Abschnitt und der ersten Achse (Achse für die Zeit) hervorgehoben gekennzeichnet. Diese Darstellung ermöglicht es einem Betrachter, rasch und ergonomisch die Fehler-Zeiträume zu identifizieren, und das auch auf einem relativ kleinen Bildschirm, beispielsweise auf einem Smartphone oder Tablet, oder falls mehrere Anlagen gleichzeitig überwacht werden. In vielen Fällen kann der Betrachter diese Darstellung auf einem relativ kleinen Bildschirm in der Nähe der Abgasreinigungsanlage betrachten.

Die folgende Ausgestaltung spezifiziert mindestens einen Sensor der ersten Überwachungseinheit näher. Möglich ist, dass jeder Sensor der ersten Überwachungseinheit auf diese Weise aufgebaut ist, zumindest jeder Sensor, der einen Ammoniak-Gehalt zu messen vermag.

Gemäß dieser Ausgestaltung umfasst der Sensor eine Sensorzelle mit einer Messkammer. Die Sensorzelle vermag den Ammoniak-Gehalt in einer Gasprobe zu messen, wobei diese Gasprobe sich in der Messkammer befindet. Weiterhin umfasst der Sensor eine röhrenförmige Zuführeinheit. Diese Zuführeinheit erstreckt sich entlang einer Längsachse. Bei einem Einsatz der Überwachungs-Anordnung ist die Zuführeinheit senkrecht oder schräg unterhalb der Messkammer angeordnet, und daher ist die Längsachse vertikal oder schräg im Raum angeordnet.

Weiterhin umfasst der Sensor ein Heizelement. Das Heizelement vermag das Innere der Zuführeinheit zu erhitzen. Durch die Erhitzung vermag das Heizelement zu bewirken, dass in der Zuführeinheit eine Konvektionsströmung (Kamineffekt) erzeugt wird. Diese Konvektionsströmung fördert eine Gasprobe aus der Umgebung durch die Zuführeinheit hindurch senkrecht oder schräg nach oben in die Messkammer.

Diese Ausgestaltung erspart die Notwendigkeit, eine Pumpe oder eine sonstige Fluidfördereinheit vorzusehen, um eine Gasprobe in die Messkammer zu fördern. Verglichen mit einer Fluidfördereinheit verbraucht das Heizelement in der Regel weniger elektrische Energie. Dies ist insbesondere dann von Vorteil, wenn der Sensor wenigstens zeitweise nicht mit einem stationären Spannungsversorgungsnetz verbunden oder verbindbar ist und daher eine eigene Spannungsversorgungseinheit umfasst. Außerdem hat das Heizelement im Gegensatz zu einer Fluidfördereinheit kein bewegtes Teil. Ein bewegtes Teil verschleißt in der Regel schneller als das Heizelement und kann Vibrationen verursachen. Andererseits fördert die erzeugte Konvektionsströmung eine Gasprobe schneller in die Messkammer, als wenn die Gasprobe ausschließlich durch Diffusion in die Messkammer gelangen würde.

In einer Ausgestaltung ist mindestens einem Sensor der ersten Überwachungseinheit ein röhrenförmiges Schutzelement zugeordnet. Dieses Schutzelement erstreckt sich entlang einer Längsachse. Bei einem Einsatz der Überwachungs-Anordnung ist die Längsachse des Schutzelements vertikal oder schräg angeordnet. Der Sensor ist im Inneren des Schutzelements angeordnet. Eine Gasprobe fließt von einer Stirnfläche des Schutzelements zum Sensor. Diese Ausgestaltung lässt sich mit der gerade beschriebenen Ausgestaltung kombinieren, bei der eine Konvektionsströmung eine Gasprobe in die Messkammer fördert.

Die Ausgestaltung mit dem röhrenförmigen Schutzelement reduziert die Gefahr, dass eine Luftströmung in der Nähe des Sensors ein Messergebnis verfälscht. Die Mantelfläche des Schutzelements reduziert den Einfluss der Luftströmung auf den Vorgang, dass eine Gasprobe in die Messkammer des Sensors eintritt. In aller Regel tritt nämlich zwischen der Luftströmung und der Längsachse des Schutzelements ein ausreichend großer Winkel auf, weil zumindest im Freien eine Luftströmung in der Regel annähernd waagerecht fließt.

In einer bevorzugten Ausgestaltung umfasst die erste Überwachungseinheit zusätzlich eine erste Kommunikationseinheit. Die Überwachungs-Anordnung umfasst zusätzlich einen stationären oder mobilen Zentralrechner. Der Zentralrechner ist räumlich von der ersten Überwachungseinheit beabstandet angeordnet, bevorzugt auch außerhalb eines Gebäudes oder sonstigen Bereichs, in dem die oder eine zu überwachende Abgasreinigungsanlage untergebracht ist. Falls die Überwachungs-Anordnung gleichzeitig mehrere Abgasreinigungsanlage überwacht, ist der Zentralrechner bevorzugt von jeder überwachten Abgasreinigungsanlage beabstandet. Die Auswertungseinheit ist ein Bestandteil des Zentralrechners.

Zwischen jedem Sensor und der ersten Kommunikationseinheit ist jeweils eine Datenverbindung dauerhaft oder wenigstens zeitweise hergestellt oder lässt sich herstellen. Außerdem ist eine Datenverbindung zwischen der ersten Kommunikationseinheit und den Zentralrechner dauerhaft oder wenigstens zeitweise hergestellt oder lässt sich herstellen. Bevorzugt wird diese Datenverbindung mithilfe von Funkwellen, also drahtlos, realisiert.

Die erste Kommunikationseinheit vermag das jeweilige Signal jedes Sensors zu empfangen und zu verarbeiten. Die erste Kommunikationseinheit vermag für jedes empfangene Signal jeweils eine Nachricht zu generieren und zu bewirken, dass die generierte Nachricht an den Zentralrechner übermittelt wird. Die Nachricht umfasst eine Information über den Ammoniak-Gehalt, den derjenige Sensor gemessen hat, von dem das Signal stammt.

Möglich ist, mit Hilfe der erfindungsgemäßen Überwachungs-Anordnung gleichzeitig mehrere Abgasreinigungsanlagen aus der Ferne zu überwachen. Bei dieser Anwendung erreichen daher in der Regel Signale von unterschiedlichen Überwachungseinheiten den Zentralrechner. Die nachfolgend beschriebene Ausgestaltung erleichtert es, dass der Zentralrechner jedes empfangene Signal der richtigen Überwachungseinheit und damit der richtigen überwachten Abgasreinigungsanlage zuordnet. Die nachfolgend beschriebene Ausgestaltung ermöglicht es, erfordert es aber nicht, dass im Zentralrechner eine rechnerverfügbare Liste vorgehalten und laufend aktualisiert wird, wobei diese Liste alle aktuell überwachten Abgasreinigungsanlagen kennzeichnet. Vielmehr kann sich eine weitere Kommunikationseinheit auch im laufenden Betrieb der Überwachungs-Anordnung beim Zentralrechner "anmelden".

Gemäß dieser Ausgestaltung umfasst die erste Kommunikationseinheit der ersten Überwachungseinheit einen Datenspeicher. Oder die erste Kommunikationseinheit hat dauerhaft oder wenigstens zeitweise Lesezugriff auf einen solchen Datenspeicher. Im Datenspeicher sind folgende Informationen abgespeichert:

- einerseits eine eindeutige Kennung der ersten Kommunikationseinheit und
- andererseits für jeden Sensor jeweils eine eindeutige Kennung.

Die eindeutige Kennung der ersten Kommunikationseinheit unterscheidet die erste Kommunikationseinheit von jeder anderen Kommunikationseinheit, die wenigstens zeitweise in einer Datenverbindung mit dem Zentralrechner steht. Die eindeutige Kennung eines Sensors unterscheidet diesen Sensor von jedem anderen Sensor der ersten Überwachungseinheit. Möglich, aber nicht erforderlich ist, dass die eindeutige Kennung eines Sensors diesen Sensor auch von jedem Sensor einer anderen Überwachungseinheit der Überwachungs-Anordnung unterscheidet.

Gemäß der gerade beschriebenen Ausgestaltung empfängt die erste Kommunikationseinheit von jedem Sensor der ersten Überwachungseinheit jeweils ein Signal, wobei dieses Signal von diesem Sensor generiert worden ist und eine Information über den von diesem Sensor gemessenen Ammoniak-Gehalt umfasst. Die erste Kommunikationseinheit generiert eine Nachricht, wobei diese Nachricht eine Information über den gemessenen Ammoniak-Gehalt umfasst. Die erste Kommunikationseinheit bewirkt, dass diese Nachricht an den Zentralrechner übermittelt wird. Gemäß der gerade beschriebenen Ausgestaltung generiert die erste Kommunikationseinheit die Nachricht so, dass die Nachricht zusätzlich die Kennung der ersten Kommunikationseinheit und die Kennung des Sensors, der den Ammoniak-Gehalt gemessen und das Signal generiert hat, umfasst.

Optional ist im Datenspeicher der ersten Überwachungseinheit mindestens eine der folgenden Informationen abgespeichert:

- eine Kennung dafür, durch welches Wirkprinzip die Abgasreinigungsanlage Ammoniak entfernt, und / oder
- eine Kennung dafür, wie viele Rohgas-Sensoren und wie viele Reingas-Sensoren die erste Überwachungseinheit umfasst, also Kennungen für zwei Anzahlen.

Die Kennung für das Wirkprinzip umfasst insbesondere eine Kennung, ob die Abgasreinigungsanlage rein chemisch oder aber vollständig oder wenigstens teilweise biologisch arbeitet.

Gemäß der gerade beschriebenen Ausgestaltung generiert die erste Kommunikationseinheit also eine Nachricht, wobei diese Nachricht

- eine Kennung der ersten Überwachungseinheit,
- eine Information über den Ammoniak-Gehalt, den ein Sensor der ersten Überwachungseinheit gemessen hat und
- eine Kennung des Sensors

umfasst. Optional umfasst die generierte Nachricht zusätzlich eine Kennung dafür, wie viele Rohgas-Sensoren und wie viele Reingas-Sensoren die erste Überwachungseinheit umfasst, und / oder eine Kennung für das Wirkprinzip der Abgasreinigungsanlage. Diese Ausgestaltung erspart die Notwendigkeit, für jede überwachte Abgasreinigungsanlage jeweils eine eigene Auswertungseinheit auf dem Zentralrechner vorsehen zu müssen. Vielmehr reicht es in der Regel aus, mehrere Varianten der Auswertungseinheit vorrätig zu halten, wobei jede Variante sich auf jeweils eine Anzahl von Rohgas-Sensoren und eine Anzahl von Reingas-Sensoren bezieht. Bei M Rohgas-Sensoren und N Reingas-Sensoren umfasst das Auswertungsprogramm mindestens M*N verschiedene Varianten. Die jeweils zutreffende Variante wird ausgewählt und eingesetzt.

Optional hängt es zusätzlich vom Wirkprinzip der überwachten Abgasreinigungsanlage ab, welche Variante die Auswertungseinheit anwendet, um die Signale der Sensoren derjenigen Überwachungseinheit, die dieser Abgasreinigungsanlage zugeordnet ist, auszuwerten. Bei K verschiedenen möglichen Wirkprinzipien gibt es insgesamt M*N*K verschiedene Varianten. Im Datenspeicher der ersten Überwachungseinheit ist in einer Realisierungsform eine Kennung dafür abgespeichert, welches Wirkprinzip die zugeordnete Abgasreinigungsanlage anwendet. Diese Kennung wird an den Zentralrechner übermittelt und von der Auswertungseinheit ausgewertet. Bevorzugt umfasst die Nachricht, welche die erste Überwachungseinheit erzeugt und welche ein Signal eines Sensors umfasst, zusätzlich die Kennung des tatsächlich angewendeten Wirkprinzips.

Nachfolgend wird eine weitere Ausgestaltung beschrieben. Diese weitere Ausgestaltung lässt sich kombinieren mit der Ausgestaltung, bei der eine Kennung des angewendeten Wirkprinzips übermittelt und ausgewertet wird.

Gemäß der weiteren Ausgestaltung wird das Rohgas durch eine Reinigungsflüssigkeit hindurchgeleitet, wobei die Reinigungsflüssigkeit zur überwachten Abgasreinigungsanlage gehört und bevorzugt eine wässrige Lösung ist. Wenn das Rohgas durch die Reinigungsflüssigkeit hindurch geleitet wird, nimmt die Reinigungsflüssigkeit aus dem Rohgas Ammoniak oder eine chemische Verbindung von Ammoniak mit weiteren Bestandteilen auf und reduziert dadurch den Ammoniak-Gehalt. Beispielsweise enthält die Reinigungsflüssigkeit Schwefelsäure, und die Schwefelsäure und Ammoniak im Rohgas bewirken eine chemische Reaktion.

Die erste Überwachungseinheit umfasst bei dieser Realisierungsform einer Abgasreinigungsanlage einen Leitfähigkeits-Sensor und / oder einen pH-Wert-Sensor. Der Leitfähigkeits-Sensor vermag die elektrische Leitfähigkeit der Reinigungsflüssigkeit zu messen und ein Signal zu generieren. Das Signal umfasst eine Information über die gemessene elektrische Leitfähigkeit. Die Messposition des Leitfähigkeits-Sensors befindet sich bevorzugt flussabwärts von dem Bereich, in der das Rohgas durch die Reinigungsflüssigkeit geleitet wird. Der pH-Wert-Sensor vermag den pH-Wert der Reinigungsflüssigkeit zu messen und ein Signal mit dem gemessenen pH-Wert zu generieren. Bevorzugt befindet die Messposition des pH-Wert-Sensors sich flussabwärts von der Abgasreinigungsanlage, beispielsweise in einem Behälter, in dem die Reinigungsflüssigkeit gesammelt wird. Das generierte Signal umfasst eine Information über den gemessenen pH-Wert.

Die beiden Signale werden von der ersten Überwachungseinheit an den Zentralrechner übermittelt, bevorzugt als Teil einer Nachricht, und von der Auswertungseinheit verarbeitet. Erfindungsgemäß detektiert die Auswertungseinheit jeden Zeitraum, in dem die Gütefunktion für die Abgasreinigungsanlage durchgehend einen Funktionswert annimmt, der kleiner ist als eine vorgegebene untere Schranke. Gemäß der gerade beschriebenen Ausgestaltung detektiert die Auswertungseinheit zusätzlich jeden Zeitraum, in dem durchgehend mindestens eines der folgenden beiden Ereignisse vorliegt:

- Die elektrische Leitfähigkeit der Reinigungsflüssigkeit ist größer als eine vorgegebene obere Schranke.
- Der pH-Wert der Reinigungsflüssigkeit ist kleiner als eine vorgegebene untere Schranke, d.h. die Reinigungsflüssigkeit ist relativ sauer.

Die Auswertungseinheit klassifiziert und verwendet jeden Zeitraum mit einer kleinen Gütefunktionen und / oder einer großen elektrischen Leitfähigkeit und / oder einem kleinen pH-Wert als einen Fehler-Zeitraum.

Ein Hintergrund dieser Ausgestaltung ist der folgende: Bei einer hohen elektrischen Leitfähigkeit kann in der Reinigungsflüssigkeit ein chemischer Zersetzungsprozess auftreten, nachdem die Reinigungsflüssigkeit das Ammoniak aufgenommen hat. Als Folge können in relevantem Umfang Stickoxide ($NO_X$) aus der Reinigungsflüssigkeit entweichen und in die Umgebung gelangen. Ein kleiner pH-Wert kann ebenfalls ein Indiz dafür sein, dass Stickoxide in die Umgebung gelangen. Dass Stickoxide in die Umgebung gelangen, ist ein unerwünschtes Ereignis. Daher wird ein Zeitraum, in dem durchgehend dieses unerwünschte Ereignis auftritt, ebenfalls als ein Fehler-Zeitraum behandelt. Diese Ausgestaltung lässt sich mit einem Sensor für Stickoxide kombinieren, erspart aber die Notwendigkeit, einen Sensor für Stickoxide vorzusehen.

Nachfolgend wird eine Realisierungsform der gerade beschriebenen Kombination der beiden Ausgestaltungen beschrieben, nämlich dass einerseits eine Kennung für das angewendete Wirkprinzip verwendet wird und andererseits die elektrische Leitfähigkeit und der pH-Wert gemessen und ausgewertet werden. Abgespeichert und ausgewertet wird eine Kennung, die mindestens die folgenden beiden Wirkprinzipien unterscheidet:

- Die Abgasreinigungsanlage arbeitet rein chemisch.
- Die Abgasanlage arbeitet vollständig oder wenigstens teilweise biologisch.

Das oben beschriebene Problem, dass Stickoxide bei einer hohen elektrischen Leitfähigkeit oder einem geringen pH-Wert entweichen, tritt nur bei einer biologisch arbeitenden Abgasreinigungsanlage auf. Das Auswertungsprogramm verwendet daher nur dann die gemessene elektrische Leitfähigkeit und den gemessenen pH-Wert, wenn zusätzlich die Kennung übermittelt wird, dass die Abgasreinigungsanlage biologisch arbeitet.

Eine nachfolgend beschriebene bevorzugte Ausgestaltung ermöglicht es insbesondere, jede Datenverbindung der Überwachungs-Anordnung zu überprüfen. Gemäß dieser Ausgestaltung umfasst die Überwachungs-Anordnung für den oder jeden Rohgas-Sensor und den oder jeden Reingas-Sensor der ersten Überwachungseinheit jeweils einen Signalgeber. Jeder Signalgeber vermag einen Wert für einen Ammoniak-Gehalt zu erfassen, wobei dieser Wert diesem Signalgeber von einem Benutzer und / oder von der Auswertungseinheit oder einem sonstigen Bestandteil der Überwachungs-Anordnung vorgegeben worden ist. Jeder Signalgeber vermag jeweils ein Signal zu generieren, welches eine Information über denjenigen Wert für einen Ammoniak-Gehalt umfasst, der diesem Signalgeber vorgegeben und vom Signalgeber erfasst worden ist. In einer Realisierungsform umfasst das generierte Signal weiterhin eine Kennung des Signalgebers, wobei diese Kennung diesen Signalgeber von dem oder jedem anderen Signalgeber sowie von jedem Ammoniak-Sensor der ersten Überwachungseinheit unterscheidet.

Die Überwachungs-Anordnung lässt sich gemäß dieser Ausgestaltung wahlweise in einem Überwachungs-Modus oder in einem Überprüfungs-Modus betreiben. Das erfindungsgemäße Überwachungs-Verfahren umfasst gemäß dieser Ausgestaltung den Schritt, dass mindestens einmal ein Überprüfungs-Verfahren durchgeführt wird. Beim Betrieb im Überprüfungs-Modus und durch das Überprüfungs-Verfahren wird die Überwachungs-Anordnung überprüft.

Beim Betrieb im Überprüfungs-Modus wird für den oder jeden Rohgas-Sensor und für den oder jeden Reingas-Sensor der ersten Überwachungseinheit jeweils ein Signalgeber verwendet. Beispielsweise ist jeder Rohgas-Sensor und jeder Reingas-Sensor durch jeweils ein Signalgeber ersetzt. Das Überprüfungs-Verfahren umfasst in einer Ausgestaltung den Schritt, dass der oder jeder Rohgas-Sensor und der oder jeder Reingas-Sensor durch jeweils einen Signalgeber ersetzt worden ist, oder das Überprüfungs-Verfahren wird nach dieser Ersetzung durchgeführt. Möglich ist auch, dass wahlweise ein Sensor oder ein Signalgeber aktiviert wird, je nachdem, in welchem Modus die Überwachungs-Anordnung aktuell betrieben wird.

Die Überwachungs-Anordnung ist beim Betrieb im Überprüfungs-Modus dazu ausgestaltet, die folgenden Schritte durchzuführen, und das Überprüfungs-Verfahren umfasst die folgenden Schritte:

- Jeder Signalgeber generiert jeweils ein Signal. Diese Signal umfasst eine Information über den Wert des Ammoniak-Gehalts, der diesem Signalgeber vorgegeben und vom Signalgeber erfasst worden ist.
- Das jeweilige Signal jedes Signalgebers wird an die Auswertungseinheit übermittelt.
- Die Auswertungseinheit ermittelt für jeden Signalgeber der ersten Überwachungseinheit, welchen Wert eines Ammoniak-Gehalts diesem Signalgeber vorgegeben ist. Hierfür verwendet die Auswertungseinheit die empfangenen Signale der Signalgeber.
- Bevorzugt vergleicht die Auswertungseinheit für jeden Signalgeber den vorgegebenen mit dem ermittelten Wert.

Diese Ausgestaltung erleichtert es insbesondere, folgende möglichen Fehler und Störungen zu detektieren:

- Eine Datenverbindung zwischen einem Ammoniak-Sensor und damit einem Signalgeber einerseits und der Auswertungseinheit andererseits ist unterbrochen oder auf andere Weise gestört.
- Eine Signalverarbeitung auf dem Weg von einem Ammoniak-Sensor und damit von einem Signalgeber zur Auswertungseinheit arbeitet fehlerhaft.
- Ein Bestandteil der Überwachungs-Anordnung wird nicht ausreichend mit elektrischer Energie versorgt.

In einer Ausgestaltung wird mindestens einem Signalgeber, bevorzugt jedem Signalgeber, jeweils ein zeitlicher Verlauf von Werten eines Ammoniak-Gehalts vorgegeben. Mit anderen Worten: Mindestens einem Signalgeber werden nacheinander unterschiedliche Werte für einen Ammoniak-Gehalt vorgegeben. In einer Realisierungsform wird jedem Signalgeber jeweils ein Testmuster mit Werten für den Ammoniak-Gehalt vorgegeben. Bevorzugt umfasst der zeitliche Verlauf einerseits den Wert null und andererseits den maximal möglichen Wert für einen Ammoniak-Gehalt, den der jeweils ersetzte Ammoniak-Sensor noch zu messen vermag, und / oder der in der Realität auftreten kann. Der maximale Wert liegt bevorzugt zwischen 90% und 100%.

Die Ausgestaltung mit dem vorgegebenen zeitlichen Verlauf erleichtert es einerseits, den Überwachungs-Modus vom Überprüfungs-Modus unterscheiden, insbesondere dann, wenn der vorgegebene zeitliche Verlauf bei einem Betrieb der überwachten Abgasreinigungsanlage in der Realität nicht vorkommen kann. Andererseits erleichtert es diese Ausgestaltung zusätzlich oder besser als ohne diese Ausgestaltung, folgende Störungen zu detektieren:

- Bei einigen Werten für einen Ammoniak-Gehalt arbeitet eine Signalverarbeitung fehlerhaft.
- Der Schritt, ein Signal vom Signalgeber und damit vom ersetzten Ammoniak-Sensor zur Auswertungseinheit zu übermitteln, ist einer erheblichen Verzögerung unterworfen.

Die Erfindung betrifft weiterhin ein System, wobei dieses System mindestens eine Abgasreinigungsanlage, optional mehrere Abgasreinigungsanlagen, und eine erfindungsgemäße Überwachungs-Anordnung umfasst. Die Überwachungs-Anordnung vermag die oder jede Abgasreinigungsanlage des Systems zu überwachen. Jeder überwachten Abgasreinigungsanlage ist jeweils eine Überwachungseinheit der Überwachungs-Anordnung zugeordnet.

Im Folgenden wird die Erfindung anhand eines Ausführungsbeispiels beschrieben. Hierbei zeigt

Figur 1    schematisch eine Ausgestaltung der erfindungsgemäßen Überwachungs-Anordnung mit einer ersten Überwachungseinheit, die zwei Rohgas-Sensoren und zwei Reingas-Sensoren umfasst;

Figur 2    schematisch eine Ausgestaltung mit der ersten und einer weiteren Überwachungseinheit, die einen Rohgas-Sensor und zwei Reingas-Sensoren umfasst;

Figur 3    in einer Querschnittsdarstellung einen Teil einer Ausführungsform des Ammoniak-Sensors;

Figur 4    in einer perspektivischen Darstellung den Ammoniak-Sensor von Figur 3;

Figur 5    das Prinzip der elektrochemisch arbeitenden Sensorzelle;

Figur 6    in einer Seitenansicht eine beispielhafte Anordnung mit einem Rohgas-Sensor, einem Reingas-Sensor und weiteren Sensoren;

Figur 7    beispielhaft den jeweiligen zeitlichen Verlauf des Signals eines Rohgas-Sensors und zweier Reingas-Sensoren;

Figur 8    beispielhaft, wie das Ergebnis der Überwachung in einem Zeitraum übersichtlich dargestellt wird;

Figur 9    beispielhaft, wie das jeweilige Ergebnis der Überwachung in sechs Zeiträumen übersichtlich dargestellt wird;

Figur 10    beispielhaft eine summarische Darstellung für zwölf Monate;

Figur 11    die Ausgestaltung von Figur 1 mit Signalgebern anstelle von Ammoniak-Sensoren.

Im Ausführungsbeispiel wird die Erfindung für eine Abgasreinigungsanlage in einem Gebäude angewendet. Eine derartige Abgasreinigungsanlage vermag Ammoniak ($NH_3$) aus einem Gasgemisch zu beseitigen und dadurch den Gehalt von Ammoniak in diesem Gasgemisch zu reduzieren. Das Gasgemisch stammt im Ausführungsbeispiel aus einem Tierzuchtbetrieb. Das Gasgemisch kann auch z.B. in einer Anlage zur Aufbereitung von Klärschlamm oder in einer Produktionsanlage für bestimmte chemische Verbindungen oder für Nahrungsmittel entstehen. Vor der Reinigung enthält das Gasgemisch typischerweise eine Ammoniak-Gehalt zwischen 1 und 1000 ppm (parts per million).

Gemäß einer Ausgestaltung arbeitet eine derartige Abgasreinigungsanlage biologisch. Mikroben zersetzen das Ammoniak im Gasgemisch. Gemäß einer anderen Ausgestaltung arbeitet die Abgasreinigungsanlage nach einem chemischen Wirkprinzip. Dies bedeutet allgemein: Das Gasgemisch wird einer chemischen Reaktion unterzogen, insbesondere indem dem Gasgemisch eine chemische Substanz zugesetzt wird. Durch die chemische Reaktion wird das Ammoniak umgewandelt, und das Ergebnis der Umwandlung liefert Substanzen, die für Menschen weniger schädlich als Ammoniak oder sogar unschädlich sind.

Eine Realisierungsform eines chemischen Wirkprinzips ist die folgende: Das Gasgemisch umfassend Ammoniak wird in der chemisch arbeitenden Abgasreinigungsanlage in Kontakt mit Schwefelsäure ($H_2SO_4$) gebracht. Durch die chemische Reaktion $H_2SO_4 + 2\,NH_3 \rightarrow (NH_4)_2SO_4$ wird Ammoniumsulfat gebildet. Dieses Ammoniumsulfat ist bei üblicher Umgebungstemperatur nicht gasförmig und lässt sich als Dünger oder auch als Nährsubstanz für Mikroben verwenden.

Nachfolgend werden die Begriffe "Rohgas" und "Reingas" verwendet. Der Abgasreinigungsanlage wird das Rohgas zugeführt, und Reingas verlässt die Abgasreinigungsanlage. Das Rohgas enthält Ammoniak oder kann wenigstens Ammoniak enthalten. Falls die Abgasreinigungsanlage korrekt arbeitet, ist der Ammoniak-Gehalt im Reingas deutlich geringer als im Rohgas. Idealerweise enthält das Reingas überhaupt kein Ammoniak mehr.

"Deutlich geringer" bedeutet genauer gesagt: Die Abgasreinigungsanlage arbeitet korrekt, falls eine vorgegebene Gütefunktion für die Abgasreinigungsanlage oberhalb einer vorgegebenen unteren Schranke liegt.

Vorgegeben sind also in einer rechnerauswertbaren Form eine Gütefunktion und eine untere Schranke für die Gütefunktion. Die Gütefunktion ist umso größer, je kleiner bei konstanten Ammoniak-Gehalt im Rohgas der Ammoniak-Gehalt im Reingas ist. Beispielsweise ist die Gütefunktion gleich einem Reinigungsgrad $Rg = Rg(Amm_{rein}, Amm_{roh}) = 1 - Amm_{rein} / Amm_{roh}$, wobei $Amm_{rein}$ der Ammoniak-Gehalt im Reingas und $Amm_{roh}$ der Ammoniak-Gehalt im Rohgas sind. In einer Alternative wird als Gütefunktion ein Reinigungsgrad $Rg = \Delta / Amm_{roh}$ verwendet, wobei $\Delta = Amm_{roh} - Amm_{rein}$ gilt. In beiden Ausgestaltungen nimmt die Gütefunktion einen Wert zwischen 0 und 1 an, wobei der Wert 1 bedeutet, dass die Abgasreinigungsanlage das Ammoniak vollständig aus dem Rohgas beseitigt hat, d.h. dass $Amm_{rein} = 0$ gilt.

Durch die Erfindung wird die Abgasreinigungsanlage automatisch daraufhin überprüft, ob sie im Sinne der obigen Definition korrekt arbeitet oder nicht. Die erfindungsgemäße Anordnung umfasst m Rohgas-Sensoren und n Reingas-Sensoren. Hierbei sind m >= 1 und n >= 1 zwei Anzahlen, die miteinander übereinstimmen können oder voneinander abweichen können. Die Sensoren können alle nach dem gleichen Messprinzip arbeiten oder mindestens zwei unterschiedliche Messprinzipien anwenden. Der oder jeder Rohgas-Sensor misst den Ammoniak-Gehalt im Rohgas, ist also flussaufwärts von der Abgasreinigungsanlage angeordnet. Der oder jeder Reingas-Sensor misst den Ammoniak-Gehalt im Reingas, ist also flussabwärts von der Abgasreinigungsanlage angeordnet. Insbesondere aus folgenden Gründen ist es in vielen Fällen vorteilhaft, mindestens zwei Rohgas-Sensoren und / oder mindestens zwei Reingas-Sensoren zu verwenden:

- Der Ammoniak-Gehalt im Rohgas oder der im Reingas zu einem Zeitpunkt kann im Raum variieren.
- Ein Sensor kann vollständig ausfallen.
- Ein Sensor kann einen Messwert mit einem erheblichen Messfehler liefern, in der Regel einen zu kleinen Messwert.

Die Erfindung lässt sich auch mit nur einem Rohgas-Sensor und / oder nur einem Reingas-Sensor realisieren.

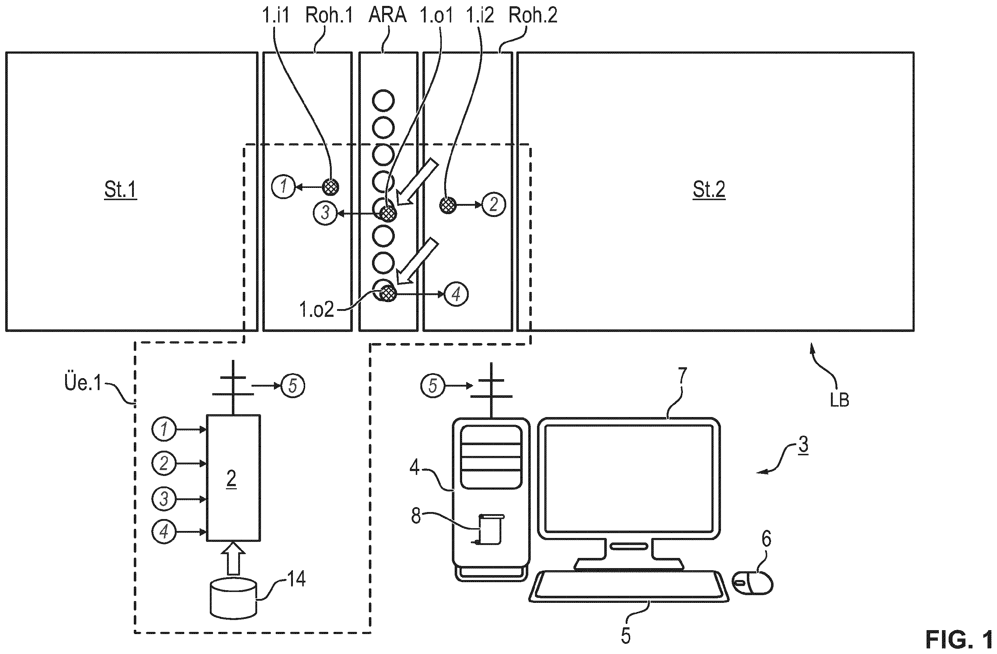

Figur 1 zeigt beispielhaft und schematisch eine Ausgestaltung der erfindungsgemäßen Überwachungs-Anordnung mit einer ersten Überwachungseinheit Ue.1 und einem Zentralrechner 3. Bestandteile der erfindungsgemäßen Überwachungs-Anordnung werden mit Ziffern bezeichnet, sonstige Gegenstände mit Buchstaben.
In der gezeigten Anwendung wird die Abgasreinigungsanlage ARA in einem landwirtschaftlichen Betrieb LB eingesetzt, der zwei Ställe St.1, St.2 umfasst. In beiden Ställen St.1, St.2 werden Tiere gehalten, und daher kann in jedem Stall St.1, St.2 Ammoniak entstehen. Die erste Überwachungseinheit Ue.1 ist der Abgasreinigungsanlage ARA zugeordnet und überwacht diese.
In einem Bereich Roh.1 kann sich Rohgas ansammeln, das aus dem Stall St.1 austritt. Entsprechend kann sich in einem Bereich Roh.2 Rohgas ansammeln, das aus dem Stall St.2 austritt. Die Abgasreinigungsanlage ARA vermag sowohl aus dem Rohgas des Bereichs Roh.1 als auch aus dem Rohgas des Bereichs Roh.2 Ammoniak zu entfernen.
Ein erster Rohgas-Sensor 1.i1 ist im Bereich Roh.1 angeordnet und vermag den Ammoniak-Gehalt in dem Gasgemisch, das sich im Bereich Roh.1 befindet, zu messen. Entsprechend ist ein zweiter Rohgas-Sensor 1.i2 im Bereich Roh.2 angeordnet und vermag den Ammoniak-Gehalt in dem Gasgemisch, das sich im Bereich Roh.2 befindet, zu messen. Die beiden Rohgas-Sensoren 1.i1, 1.i2 messen den Ammoniak-Gehalt an zwei verschiedenen Messpositionen, und der Ammoniak-Gehalt kann nicht nur über der Zeit, sondern auch von Messposition zu Messposition variieren.
Flussabwärts von der Abgasreinigungsanlage ARA sind zwei Reingas-Sensoren 1.o1, 1.o2 angeordnet. Diese Sensoren messen an zwei voneinander beabstandeten Messpositionen den Ammoniak-Gehalt im Reingas.
Jeder Sensor 1.i1, 1.i2, 1.o1, 1.o2 generiert jeweils ein Signal, welches eine Information über den vom Sensor 1.i1, 1.i2, 1.o1, 1.o2 jeweils gemessenen Ammoniak-Gehalt umfasst. Bevorzugt wird der Ammoniak-Gehalt in ppm (parts per million) angegeben, alternativ in Vol-% oder Gew-%. Die Sensoren 1.i1, 1.i2, 1.o1, 1.o2 übermitteln ihre Signale an eine Kommunikationseinheit 2, welche als die erste Kommunikationseinheit fungiert. In einer Ausgestaltung fragt die Kommunikationseinheit 2 die Sensoren 1.i1, 1.i2, 1.o1, 1.o2 regelmäßig ab, und als Antwort auf eine Abfrage übermittelt der abgefragte Sensor mindestens einen Signalwert - es sei denn, der abgefragte Sensor ist defekt, oder die Datenverbindung ist unterbrochen. Die Kommunikationseinheit 2 ist ebenfalls im landwirtschaftlichen Betrieb LB angeordnet. Die Signale werden per Kabel und / oder per Funkwellen von den Sensoren 1.i1, 1.i2, 1.o1, 1.o2 an die Kommunikationseinheit 2 übermittelt. Die Sensoren und die Kommunikationseinheit 2 gehören zur ersten Überwachungseinheit Ue.1, die dem landwirtschaftlichen Betrieb LB mit der Abgasreinigungsanlage ARA zugeordnet ist.
Die Kommunikationseinheit 2 hat wenigstens zeitweise Lesezugriff auf einen Datenspeicher 14. In diesem Datenspeicher 14 sind Informationen über die erste Überwachungseinheit Ue.1 und Informationen über jeden Sensor der ersten Überwachungseinheit Ue.1 abgespeichert.
Einerseits sind folgende Informationen im Datenspeicher 14 abgespeichert:

- eine eindeutige Kennung (ID) der Kommunikationseinheit 2 und damit eine eindeutige Kennung der zugeordneten und zu überwachenden Abgasreinigungsanlage ARA, wobei die Kennung ID diese Kommunikationseinheit 2 von jeder anderen Kommunikationseinheit unterscheidet, und
- eine eindeutige Kennung b oder c oder bc, welche spezifiziert, welches Reinigungsprinzip die überwachte Abgasreinigungsanlage ARA anwendet, beispielsweise ob sie biologisch oder chemisch oder auf beide Weisen Ammoniak entfernt.

Andererseits ist für jeden Sensor 1.i1, 1.i2, 1.o1, 1.o2 jeweils eine eindeutige Kennung abgespeichert. Diese eindeutige Kennung umfasst folgende Bestandteile:

- eine eindeutige Kennung i1, i2, o1, o2 für den Sensor, wobei die eindeutige Kennung diesen Sensor 1.i1, 1.i2, 1.o1, 1.o2 von jedem anderen Sensor in demselben Betrieb LB unterscheidet, und
- in einer Ausgestaltung, ob der Sensor Reingas oder Rohgas misst, also flussaufwärts oder flussabwärts von der überwachten Abgasreinigungsanlage ARA angeordnet ist.

Zur erfindungsgemäßen Überwachungs-Anordnung gehört im Ausführungsbeispiel weiterhin ein Zentralrechner 3, der folgende Bestandteile umfasst:

- eine signalverarbeitende Recheneinheit 4,
- einen Bildschirm 7,
- eine Tastatur 5 und
- eine Maus 6.

Der Zentralrechner 3 ist räumlich entfernt von dem Betrieb LB angeordnet, in dem sich die zugeordnete und überwachte Abgasreinigungsanlage ARA befindet. Die Kommunikationseinheit 2 übermittelt die Signale von den Sensoren 1.i1, 1.i2, 1.o1, 1.o2 per Kabel und / oder per Funkwellen an den Zentralrechner 3. Jedes übermittelte Signal umfasst die oben beschriebenen Informationen, also

- die eindeutige Kennung der Kommunikationseinheit 2,
- die Anzahl m,n der Sensoren der Abgasreinigungsanlage ARA,
- die Wirkungsweise der Abgasreinigungsanlage ARA und
- die oben beschriebenen eindeutigen Kennung desjenigen Sensors 1.i1, 1.i2, 1.o1, 1.o2, von dem der gemessene Ammoniak-Gehalt im Signal stammt.

Die Kommunikationseinheit 2 ermittelt diese Informationen durch einen Lesezugriff auf den Datenspeicher 14.

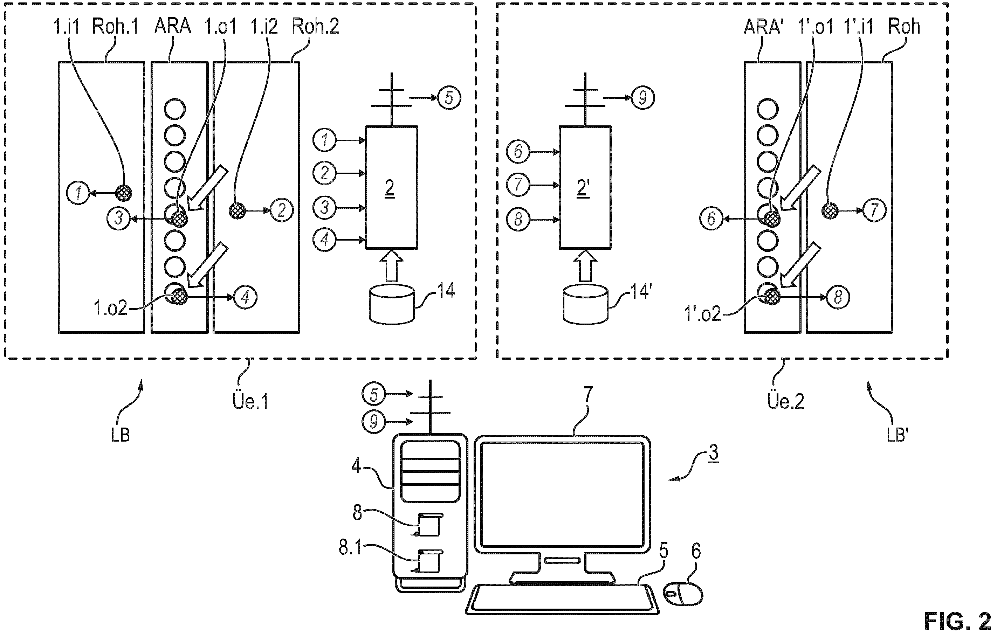

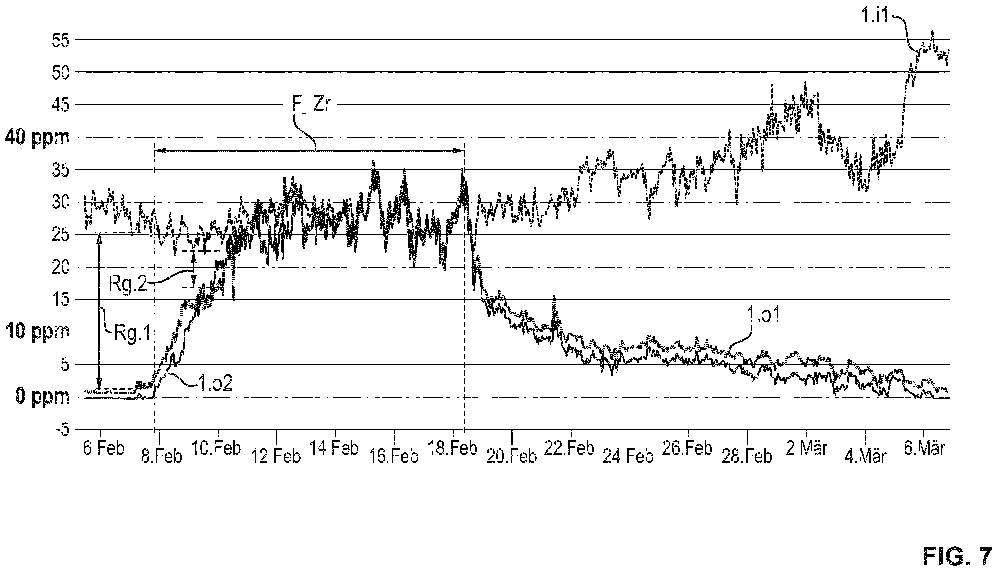

Die Recheneinheit 4 umfasst einen Prozessor und einen Datenspeicher. Im Datenspeicher ist ein Auswertungsprogramm 8 abgespeichert. Der Prozessor vermag das Auswertungsprogramm 8 auszuführen und bei der Ausführung Signale zu verarbeiten. Diese Signale hat der Zentralrechner 3 von der ersten Überwachungseinheit Ue.1 und optional von weiteren Überwachungseinheiten empfangen, vgl. Figur 2. Der Prozessor vermag abhängig von den verarbeiteten Signalen verschiedene Darstellungen zu erzeugen. Die Signalverarbeitung und die Darstellungen werden weiter unten mit Bezug auf Figur 7 bis Figur 10 beschrieben.

Im Ausführungsbeispiel ist die Auswertungseinheit auf der Recheneinheit 4 installiert und umfasst das Auswertungsprogramm 8. Möglich ist auch, dass die Auswertungseinheit oder wenigstens ein Teil der Auswertungseinheit ein Bestandteil der Kommunikationseinheit 2 ist.

Figur 2 zeigt die Abgasreinigungsanlage ARA, die erste Überwachungseinheit Ue.1 von Figur 1 sowie eine weitere Abgasreinigungsanlage ARA' und eine weitere Überwachungseinheit Ue.2 in einem weiteren landwirtschaftlichen Betrieb LB'. Die weitere Abgasreinigungsanlage ARA' beseitigt Ammoniak aus Rohgas, das sich in einem weiteren Bereich Roh ansammeln kann. Die weitere Überwachungseinheit Üe'.2 umfasst einen weiteren Rohgas-Sensor 1'.i1, zwei weitere Reingas-Sensoren 1'.o1, 1'.o2 und eine weitere Kommunikationseinheit 2'. Wenigstens zeitweise ist jeweils eine Datenverbindung zwischen einem weiteren Sensor 1'.i1, 1'.o1, 1'.o2 und der weiteren Kommunikationseinheit 2' hergestellt. Außerdem ist wenigstens zeitweise eine Datenverbindung zwischen der weiteren Kommunikationseinheit 2' und dem Zentralrechner 3 hergestellt, wobei der Zentralrechner 3 bereits mit Bezug auf Figur 1 beschrieben wurde.

In dem Beispiel, das in Figur 2 gezeigt wird, empfängt der Zentralrechner 3 sowohl Signale von der ersten Kommunikationseinheit 2 als auch Signale von der weiteren Kommunikationseinheit 2'. Jede Kommunikationseinheit 2, 2' gehört zu jeweils einer Überwachungseinheit Ue.1, Ue.2, wobei diese Überwachungseinheit Ue.1, Ue.2 außerdem jeweils m Rohgas-Sensoren und n Reingas-Sensoren umfasst. Es gilt $1 <= m <= M$ und $1 <= n <= N$, wobei M und N vorgegebene Maximal-Anzahlen von Sensoren sind und beispielsweise M gleich N gleich 4 gilt. Die Anzahlen m und n können von Überwachungseinheit Ue.1 zu Überwachungseinheit Ue.2 variieren. Das Auswertungsprogramm 8 auf der Recheneinheit 4 umfasst M*N Varianten, nämlich jeweils eine Variante für eine Überwachungseinheit mit m Rohgas-Sensoren und n Reingas-Sensoren, wobei $1 <= m <= M$ und $1 <= n <= N$.

Falls die Auswertung davon abhängt, welches Wirkprinzip die überwachte Abgasreinigungsanlage anwendet, und falls es K verschiedene Wirkprinzipien gibt, werden M*N*K verschiedene Varianten des Auswertungsprogramms bereitgestellt.

Beispielhaft wird in Figur 2 zusätzlich zum Auswertungsprogramms 8 ein Auswertungsprogramm 8.1 gezeigt. Das Auswertungsprogramm 8 ist für eine Sorte von Abgasreinigungsanlagen, beispielsweise für biologisch arbeitende, und das Auswertungsprogramm 8.1 für eine zweite Sorte, beispielsweise für chemisch arbeitende, bestimmt. Jedes Auswertungsprogramm 8, 8.1 umfasst jeweils M*N Varianten.

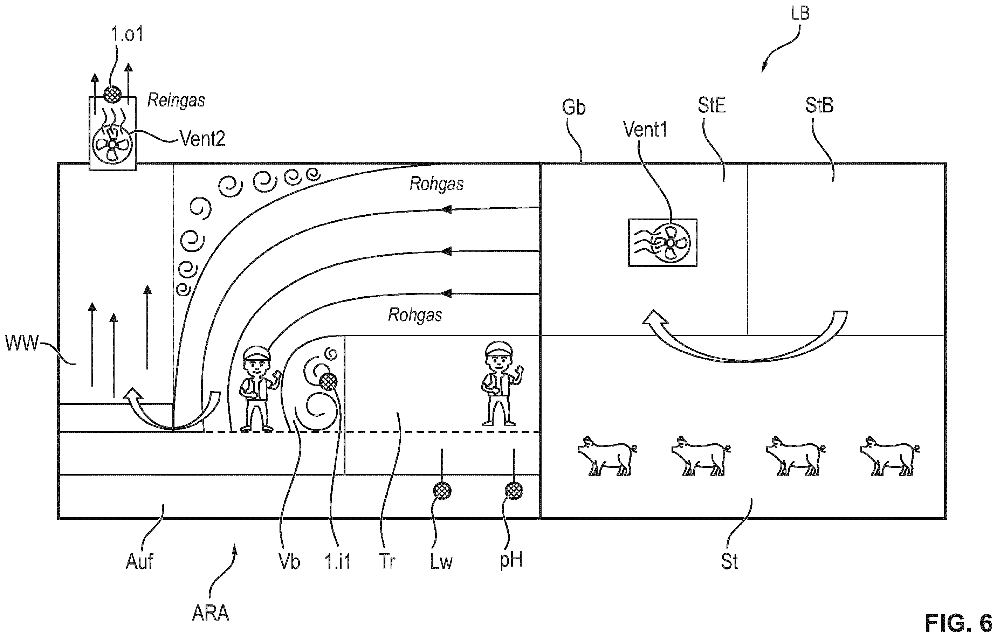

In einer anderen Realisierungsform umfasst das Auswertungsprogramm 8 jeweils zwei Varianten für eine Überwachungseinheit mit m Rohgas-Sensoren und n Reingas-Sensoren, nämlich eine Variante für eine Abgasreinigungsanlage, die Ammoniak ausschließlich chemisch beseitigt, und eine Variante für eine Abgasreinigungsanlage, die Ammoniak biologisch oder sowohl biologisch als auch chemisch beseitigt. Weiter unten wird mit Bezug auf Figur 6 erläutert, wodurch sich diese beiden Varianten voneinander unterscheiden.

Wie bereits dargelegt, führt der Prozessor der Recheneinheit 4 das Auswertungsprogramm 8 aus. Das Auswertungsprogramm 8 wertet die Signale von jeder verbundenen Kommunikationseinheit 2, 2' aus. Durch diese Auswertung ermittelt das Auswertungsprogramm 8 für jede verbundene Überwachungseinheit Ue.1, Ue.2,

- auf welche Abgasreinigungsanlage ARA, ARA' sich die Signale dieser Überwachungseinheit Ue.1, Ue.2 beziehen,
- wie viele Rohgas-Sensoren und wie viele Reingas-Sensoren diese Überwachungseinheit Ue.1, Ue.2 aufweist und
- ob die zugeordnete überwachte Abgasreinigungsanlage ARA, ARA' biologisch, chemisch oder sowohl biologisch als auch chemisch arbeitet.

Außerdem ermittelt das Auswertungsprogramm 8, von welchem Sensor ein Signal umfassend eine Information über einen Ammoniak-Gehalt stammt.

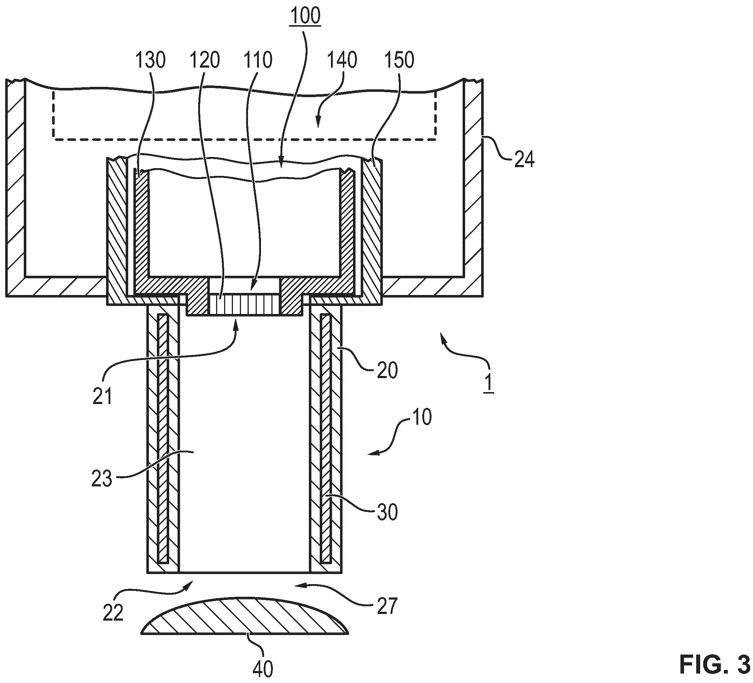

Nachfolgend wird mit Bezug auf Figur 3 bis Figur 5 eine beispielhafte Ausgestaltung eines Ammoniak-Sensors 1 beschrieben. Sowohl die beiden Rohgas-Sensoren 1.i1, 1.i2 als auch die beiden Reingas-Sensoren 1.o1, 1.o2 von Figur 1 können so aufgebaut sein wie nachfolgend beschrieben. Die nachfolgend beschriebenen Bezeichnungen "oben" und "unten" beziehen sich auf eine Orientierung des Ammoniak-Sensors 1 im regulären Betrieb.

Der Ammoniak-Sensor 1 umfasst eine Sensorzelle 100 mit einer Messkammer. Eine Wand 130 und ein poröser Schutzfilter 120 umgeben die Sensorzelle 100. Die Sensorzelle 100 misst den Ammoniak-Gehalt in einer Gasprobe, welche sich in der Messkammer mit der Wand 130 befindet. Die Sensorzelle 100 wendet mindestens eines von mehreren bekannten Messprinzipien an, um den Ammoniak-Gehalt in einem Gasgemisch zu messen.

Die Gasprobe fließt von unten durch eine Eintrittsöffnung 22 hindurch in eine röhrenförmige Zuführeinheit 10 und "beruhigt sich" in einem Bereich 23 der Zuführeinheit 10. Die beruhigte Gasprobe fließt die Zuführeinheit 10 hindurch nach oben und durch eine Austrittsöffnung 21 hindurch in einen Bereich 110 der Messkammer. Dieser Bereich wird von der Wand 130, der Austrittsöffnung 21 und einer Membrane 121 begrenzt. Oberhalb der Membrane 121 befinden sich in der Messkammer ein Elektrolyt und mehrere Elektroden. Der Schutzfilter 120 ist zwischen der Zuführeinheit 10 und dem Bereich 110 der Messkammer angeordnet.

Die Zuführeinheit 10 wird von einer Wandung 20 begrenzt. In die Wandung 20 ist ein Heizelement 30 eingesetzt. Das Heizelement 30 bewirkt eine Konvektionsströmung durch die Eintrittsöffnung 22 hindurch nach oben in die Zuführeinheit 10 (Kamineffekt). Ein mechanischer Prallschutz 40 reduziert das Risiko, dass die Konvektionsströmung Partikel in die Zuführeinheit 10 mitreißt. Zwischen dem Prallschutz 40 und der Eintrittsöffnung 22 tritt ein ringförmiger Eintrittsspalt 27 auf. In diesem Eintrittsspalt 27 wird eintretendes Gas oft verwirbelt, was zu einer geringeren räumlichen Variation des Ammoniak-Gehalts in der Gasprobe führt. Daher ist die bewirkte Verwirbelung erwünscht.

Der Ammoniak-Sensor 1 umfasst weiterhin ein Gehäuse 24, das einen Innenbereich 140 umgibt. Im Innenbereich 140 sind eine Spannungsversorgungseinheit und eine signalverarbeitende Auswerteeinheit angeordnet. Dank der eigenen Spannungsversorgungseinheit ist der Ammoniak-Sensor 1 unabhängig von einem stationären Spannungsversorgungsnetz. In das Gehäuse 24 ist unten eine Aufnahme 150 eingesetzt. Diese Aufnahme 150 hält die Zuführeinheit 10 und umgibt die Wand 130 der Sensorzelle 100.

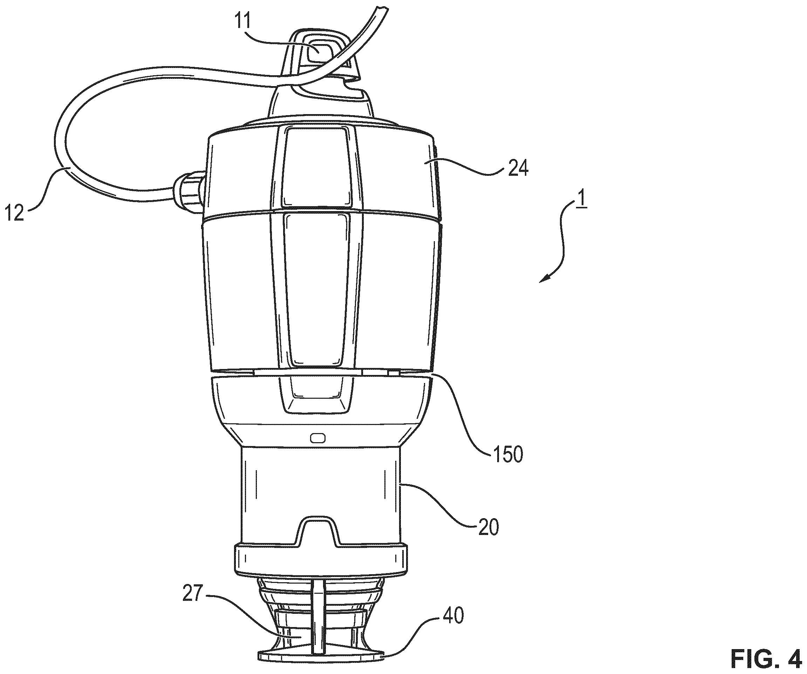

Figur 4 zeigt den Ammoniak-Sensor 1 von Figur 3 in einer Seitenansicht. Gleiche Bezugszeichen haben die gleichen Bedeutungen wie in Figur 3. Im Betrieb ist der Ammoniak-Sensor 1 so angeordnet, dass der Prallschutz 40 nach unten zeigt. Oben am Gehäuse 24 ist eine Öse 11 angebracht, mit deren Hilfe sich der Ammoniak-Sensor 1 an einen Haken hängen lässt. Außerdem ist am Gehäuse 24 ein Kabel 12 angebracht. Dank des Kabels 12 lässt sich der Ammoniak-Sensor 1 mit einem stationären Spannungsversorgungsnetz verbinden, beispielsweise um die eigene Spannungsversorgungseinheit aufzuladen.

Der oder jeder Rohgas-Sensor 1.i1, 1.i2 ist bevorzugt an einer Position angeordnet, in der das Rohgas verwirbelt wird. Beispielsweise fließt das Rohgas annähernd waagerecht über eine Fläche, erreicht eine Kante und fließt dann senkrecht oder schräg nach unten. Hierbei tritt eine Verwirbelung auf. Weil an der Messposition des Rohgas-Sensors 1.i1, 1.i2 das Rohgas verwirbelt wird, misst der Rohgas-Sensor 1.i1, 1.i2 einen Ammoniak-Gehalt, der bis zu einem gewissen Grad räumlich gemittelt ist.

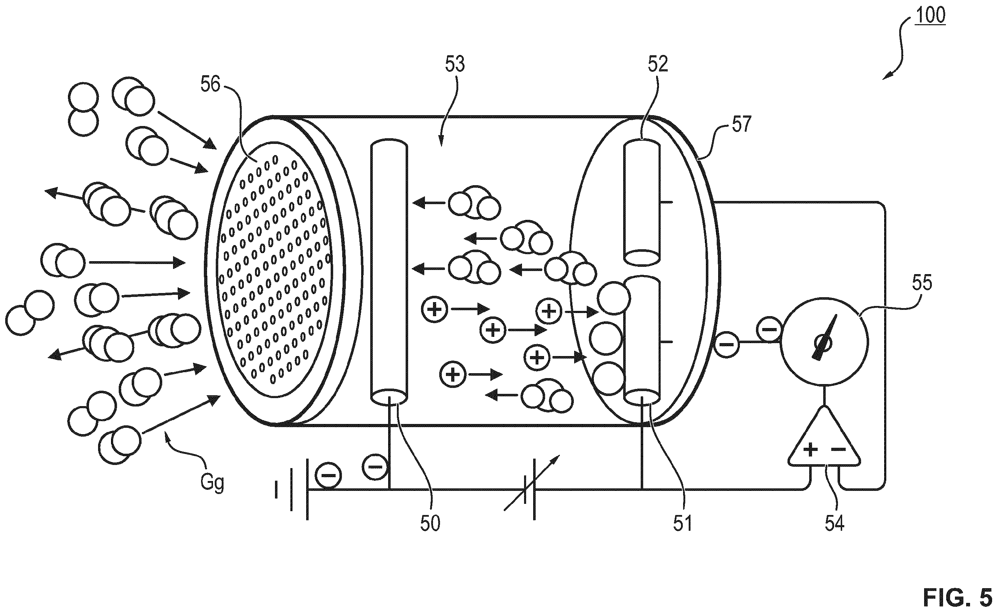

Figur 5 veranschaulicht schematisch die Wirkungsweise einer elektrochemisch arbeitenden Sensorzelle 100 eines Ammoniak-Sensors 1. Die Sensorzelle 100 arbeitet nach dem Prinzip einer Brennstoffzelle. Der Ammoniak-Gehalt in einem Gasgemisch Gg soll untersucht werden. Das Gasgemisch Gg fließt durch eine poröse Membrane 56 hindurch in eine Messkammer im Inneren eines Gehäuses 57. Im Inneren des Gehäuses befinden sich eine Messelektrode 50, eine Gegenelektrode 51 und eine Referenzelektrode 52. Zwischen der Messelektrode 50 auf der einen Seite und der Gegenelektrode 51 und der Referenzelektrode 52 auf der anderen Seite befindet sich ein ionisch leitfähiger Elektrolyt 53. Das Gasgemisch Gg erreicht den Elektrolyten 53. Wie schematisch dargestellt ist, fließt ein elektrischer Strom im Elektrolyten 53. Ein sogenannter Potentiostat 54 misst ein Maß für die insgesamt fließende elektrische Ladung. Die elektrische Ladung korreliert mit der Menge von Ammoniak in der Messkammer und somit mit dem Ammoniak-Gehalt im Gasgemisch Gg, wobei das Gasgemisch Gg sich links von der Membrane 56 befindet. Auf einer Anzeige 55 lässt sich der gemessene Ammoniak-Gehalt ablesen.

Der oder jeder Reingas-Sensor 1.o1, 1.o2 befindet sich bevorzugt schräg oberhalb der Abgasreinigungsanlage ARA, beispielsweise auf einem Dach eines Gebäudes, in welchem sich die Abgasreinigungsanlage ARA befindet. In einer Ausgestaltung saugt ein Ventilator oder eine sonstige Fluidfördereinheit das Reingas aus diesem Gebäude ab. Der oder ein Reingas-Sensor 1.o1, 1.o2 befindet sich oberhalb dieser Fluidfördereinheit und bevorzugt außerhalb und oberhalb des Gebäudes. Diese Positionierung erleichtert es, den Reingas-Sensor 1.o1, 1.o2 zu warten und zu reparieren.

Bevorzugt umgibt jeweils eine für Fluid undurchlässige Röhre jeden Sensor 1.i1, 1.i2, 1.o1, 1.o2 oder wenigstens jeden außerhalb des Gebäudes angeordneten Sensor. Die Längsachse dieser Röhre ist senkrecht angeordnet, also etwa parallel zu der Konvektionsströmung, welche vom Heizelement 30 erzeugt wird. Diese Röhre schützt den Sensor 1.i1, 1.i2, 1.o1, 1.o2 bis zu einem gewissen Grad vor mechanischen Einflüssen von außen. Außerdem schützt die Röhre den Sensor 1.i1, 1.i2, 1.o1, 1.o2 vor dem Einfluss von Wind und sonstigen Luftströmungen. Dank der Röhre wird auch bei Luftströmungen eine ausreichende Konvektionsströmung erzeugt. Die Gefahr wird verringert, dass die Luftströmungen zu Messfehlern führen.

Figur 6 zeigt in einer Seitenansicht beispielhaft einen landwirtschaftlichen Betrieb LB mit einem Stall St und einer chemisch arbeitenden Abgasreinigungsanlage ARA, die beide in einem Gebäude Gb angeordnet sind. Schematisch werden vier Schweine im Stall St gezeigt. Der Stall St wird in einem Bereich StB belüftet und in einem Bereich StE mit Hilfe eines ersten Ventilators Vent1 entlüftet. Rohgas (raw gas) aus dem Stall St, welches in der Regel einen relativ hohen Ammoniak-Gehalt umfasst, fließt aus dem Bereich StE annähernd waagerecht über ein Dach eines Technikraums Tr hinweg und sinkt dann an einer Wand des Technikraums Tr nach unten. An dieser Wand wird ein Verwirbelungsbereich Vb gebildet. Dank dieses Verwirbelungsbereichs Vb variiert der Ammoniak-Gehalt einen Zeitpunkt flussabwärts vom Verwirbelungsbereich Vb weniger stark im Raum.

Ein zweiter Ventilator Vent2 auf dem Dach des Gebäude Gb saugt Reingas (clean gas) aus dem Gebäude Gb nach oben ab. Eine Reinigungsflüssigkeit wird von oben auf eine Waschwand WW verbracht und fließt an der Waschwand WW entlang nach unten. An der Waschwand WW wird dem vorbeifließenden Rohgas Schwefelsäure ($NH_3$) zugesetzt, beispielsweise als Teil der Reinigungsflüssigkeit. Die Gabe von Schwefelsäure bewirkt die oben bereits beschriebene chemische Reaktion $H_2SO_4 + 2\,NH_3 \rightarrow (NH_4)_2SO_4$. Ein Auffangbehälter Auf schräg unterhalb der Waschwand WW nimmt die Reinigungsflüssigkeit mit dem Ammoniumsulfat $(NH_4)_2SO_4$ auf.

Ein pH-Sensor pH misst den pH-Wert der Reinigungsflüssigkeit im Auffangbehälter Auf. Ein Leitfähigkeits-Sensor LW misst die elektrische Leitfähigkeit der Reinigungsflüssigkeit im Auffangbehälter Auf. Ein Hintergrund ist folgende Begrenzung einer biologisch arbeitenden Abgasreinigungsanlage ARA: Falls die elektrische Leitfähigkeit der Reinigungsflüssigkeit im Auffangbehälter Auf größer ist als eine obere Schranke oder der pH-Wert kleiner ist als eine untere Schranke, so arbeitet die Abgasreinigungsanlage ARA möglicherweise nicht korrekt. In der Reinigungsflüssigkeit im Auffangbehälter Auf können chemische Zerfalls- oder Zersetzungsprozesse auftreten, und als Folge davon kann Stickoxid ($NO_x$) austreten. Dies ist unerwünscht. Die obere Schranke für die elektrische Leitfähigkeit ist bevorzugt kleiner als 300 mS/cm und liegt besonders bevorzugt zwischen 5 und 50 mS/cm (mS = Milli-Siemens), insbesondere zwischen 10 und 35 mS/cm. Die untere Schranke für den pH-Wert liegt bevorzugt zwischen 5 und 8.

Bevorzugt wird die Reinigungsflüssigkeit mit dem Ammoniumsulfat regelmäßig aus dem Auffangbehälter Auf abgesaugt. In einer Realisierungsform das wird Ammoniumsulfat wenigstens teilweise durch einen chemischen Prozess aus der Reinigungsflüssigkeit entfernt, beispielsweise gezielt zersetzt, und die Reinigungsflüssigkeit wird erneut zur Waschwand WW verbracht. In einer anderen Realisierungsform wird die Reinigungsflüssigkeit für einen anderen Zweck verwendet oder entsorgt, und neue Reinigungsflüssigkeit wird der Abgasreinigungsanlage zugeführt.

Bevorzugt wird durch eine Regelung (closed-loop control) sichergestellt, dass der pH-Wert der zur Waschwand WW geförderten Reinigungsflüssigkeit in einem vorgegebenen Bereich liegt. Der pH-Sensor pH misst den tatsächlichen pH-Wert der Reinigungsflüssigkeit, welche der Abgasreinigungsanlage zugeführt wird. Bei Bedarf wird der pH-Wert der zugeführten Reinigungsflüssigkeit vergrößert oder verkleinert.

In diesem Beispiel umfasst die Überwachungseinheit einen Rohgas-Sensor 1.i1, der im Verwirbelungsbereich Vb angeordnet ist, und einen Reingas-Sensor 1.o1, der oberhalb des zweiten Ventilators Vent2 und damit außerhalb des Gebäudes Gb und auf dem Dach angeordnet ist. Der Reingas-Sensor 1.o1 wird von einer senkrecht angeordneten Röhre (nicht gezeigt) umgeben. In einer Ausgestaltung empfängt die Kommunikationseinheit 2 ein Signal des pH-Sensors pH und ein Signal des Leitfähigkeits-Sensors LW und leitet diese beiden Signale weiter.

Der Zentralrechner 3 erfasst eine Benutzervorgabe, die ein Benutzer mit Hilfe der Tastatur 5 und / oder der Maus 6 vorgenommen hat. Diese Benutzervorgabe spezifiziert, welche Auswertungen der Zentralrechner 3 erzeugen und darstellen soll. Der Zentralrechner 3 erfasst die Benutzervorgabe und generiert mindestens eine Darstellung entsprechend der erfassen Benutzervorgabe. Der Zentralrechner 3 veranlasst, dass die gewünschte Darstellung in einer von einem Menschen wahrnehmbaren Form auf dem Bildschirm 7 ausgegeben wird. Um diese Darstellung zu erzeugen, empfängt der Zentralrechner 3 Signale von den verbundenen Überwachungseinheiten Ue.1, Ue.2.

Das Auswertungsprogramm 8 wertet die Signale von der jeweiligen Kommunikationseinheit 2, 2' der Überwachungseinheit Ue.1, Ue.2 aus. Wie oben dargelegt wird, umfasst das Auswertungsprogramm 8 insgesamt 2\*M\*N Varianten, wobei M die maximal mögliche Anzahl von Rohgas-Sensoren und N die maximal mögliche Anzahl von Reingas-Sensoren ist und außerdem jeweils eine Variante für eine rein chemisch arbeitende Abgasreinigungsanlage und eine Abgasreinigungsanlage, die ausschließlich oder wenigstens teilweise biologisch arbeitet, vorhanden ist.

Wie oben erwähnt, umfasst die Kennung für eine Überwachungseinheit eine Kennzeichnung dafür, wie die überwachte Abgasreinigungsanlage ARA, ARA' arbeitet, in der gezeigten Realisierungsform b oder c oder bc.

Die Variante für eine chemisch arbeitende Abgasreinigungsanlage wertet bevorzugt zusätzlich ein übermitteltes Signal eines Sensors für die elektrische Leitfähigkeit und ein übermitteltes Signal eines Sensors für den pH-Wert aus.

Durch die Auswertung ermittelt die Variante des Auswertungsprogramms 8 die jeweilige elektrische Leitfähigkeit und den jeweiligen pH-Wert und vergleicht diese beiden Werte mit jeweils einer Schranke, die weiter oben erläutert wurde. Die Variante für eine biologisch arbeitende Abgasreinigungsanlage führt bevorzugt diese Vergleiche nicht durch.

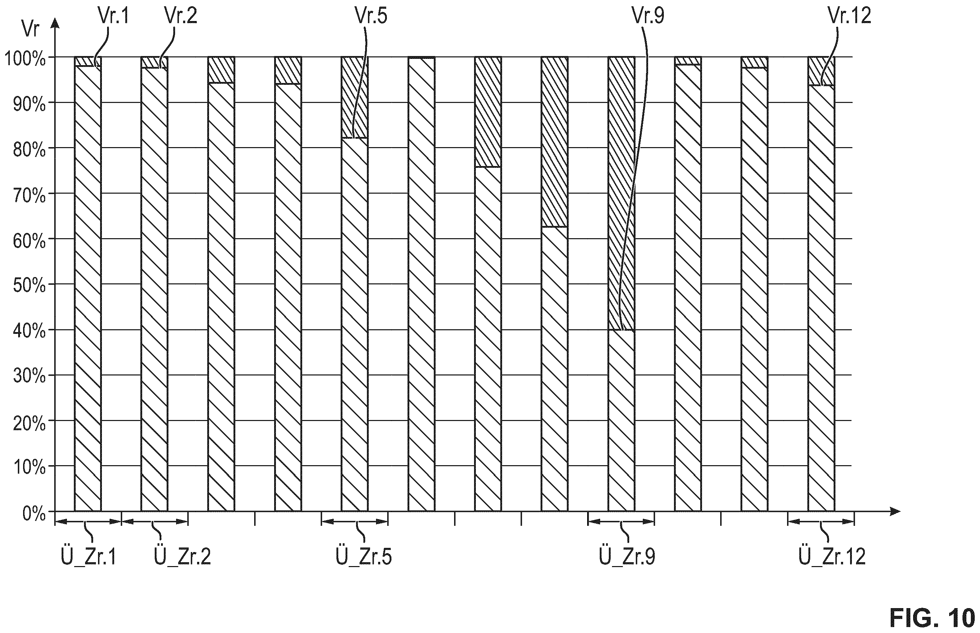

Figur 7 bis Figur 10 zeigen verschiedene Darstellungen, die sich hinsichtlich des jeweiligen Überwachungs-Zeitraums U_Zr und der Detaillierung voneinander unterscheiden. In jeder Darstellung ist auf der x-Achse die Zeit dargestellt, auf der y-Achse der Ammoniak-Gehalt (Figur 7 bis Figur 9) oder eine Verfügbarkeitsrate der überwachten Abgasreinigungsanlage (Figur 10).

Figur 7 bezieht sich auf einen Überwachungs-Zeitraum U_Zr von einem Monat Dauer. Dargestellt sind der jeweilige zeitliche Verlauf des Ammoniak-Gehalts, den der Rohgas-Sensor 1.i1 und die Reingas-Sensoren 1.o1 und 1.o2 messen, vgl. Figur 2, insgesamt also drei verschiedene zeitliche Verläufe. Das Auswertungsprogramm 8 berechnet außerdem für jeden Abtastzeitpunkt jeweils eine Gütefunktion in Form eines Reinigungsgrads Rg, beispielsweise gemäß der Rechenvorschrift

$$(1) \qquad Rg = 1 - Amm_{rein}/Amm_{roh}$$

oder auch

$$(2) \qquad Rg = (Amm_{roh} - Amm_{rein)} / Amm_{roh.}$$

Das Auswertungsprogramm 8 ermittelt jeden Fehler-Zeitraum. In einem Fehler-Zeitraum liegt der Reinigungsgrad Rg unterhalb einer vorgegebenen unteren Schranke. Die untere Schranke ist offensichtlich maximal gleich 1 und ist bevorzugt größer als 0,01, besonders bevorzugt größer als 0,6, und beträgt beispielsweise 0,9. Bei einer chemisch arbeitenden Abgasreinigungsanlage ist außerdem ein Fehler-Zeitraum ein Zeitraum, in dem die elektrische Leitfähigkeit oberhalb der vorgegebenen oberen Schranke liegt und / oder der pH-Wert unterhalb der vorgegebenen unteren Schranke liegt, selbst wenn der Reinigungsgrad Rg ausreichend groß ist.

In dem Beispiel von Figur 7 liegt der Reinigungsgrad Rg im Zeitraum F_Zr durchgehend unter 0,9 und ist außerhalb des Zeitraums größer oder gleich 0,9.

Daher ist der Zeitraum F_Zr der einzige Fehler-Zeitraum des Überwachungs-Zeitraums U_Zr, der hier eine Dauer von einem Monat aufweist.

Falls eine Überwachungseinheit Ue.1, Ue.2 mehrere Rohgas-Sensoren aufweist, ermittelt das Auswertungsprogramm 8 den Ammoniak-Gehalt im Rohgas durch eine Mittelung, z.B. eine gewichtete Mittelung, über die Messwerte, welche die Rohgas-Sensoren dieser Überwachungseinheit Ue.1, Ue.2 liefern. Das Entsprechende gilt für eine Überwachungseinheit Ue.1, Ue.2 mit mehreren Reingas-Sensoren.

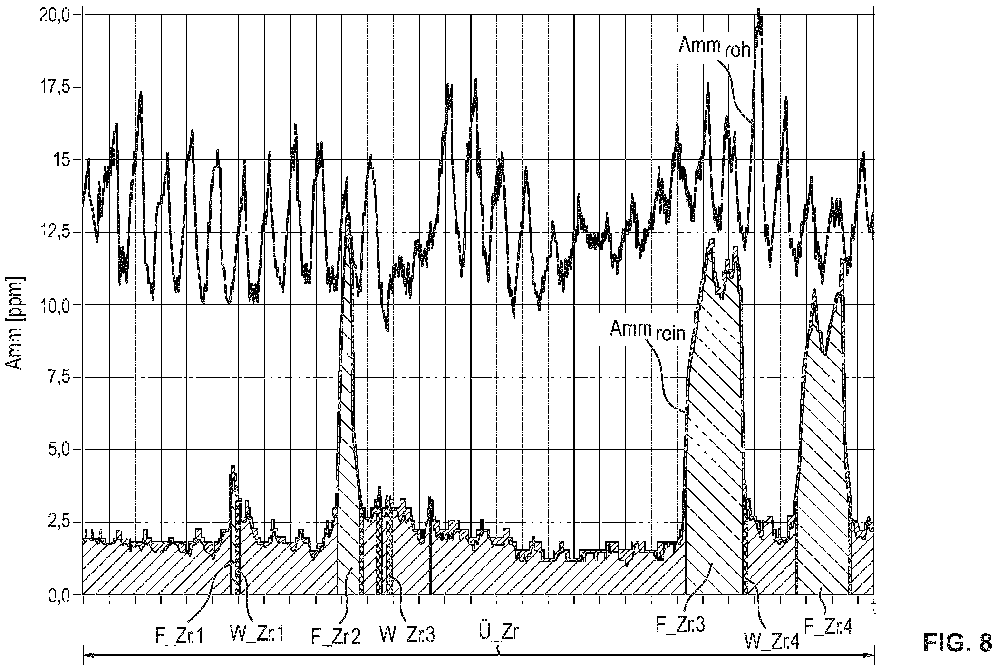

Die Darstellung von Figur 8 zeigt den zeitlichen Verlauf eines gemittelten Ammoniak-Gehalts $Amm_{roh}$ im Rohgas und den zeitlichen Verlauf eines gemittelten Ammoniak-Gehalts $Amm_{rein}$ im Reingas. In diesem Überwachungs-Zeitraum U_Zr wurden vier Fehler-Zeiträume F_Zr.1, ..., F_Zr.4 entdeckt, in denen der Reinigungsgrad Rg durchgehend kleiner ist als die vorgegebene Schranke von beispielsweise 0,9. Außerdem wurden mehrere Warnungs-Zeiträume W_Zr1, W_Zr2, ... detektiert, in denen der Reinigungsgrad Rg zwar größer als 0,9 ist, aber kleiner als eine weitere untere Schranke.

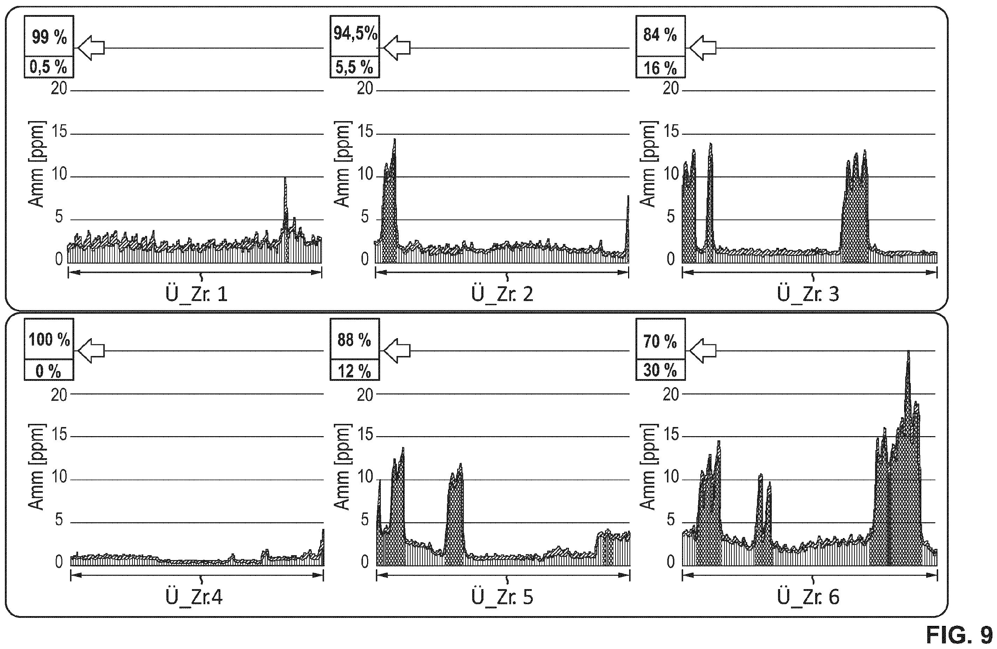

Figur 9 zeigt in einer einzigen Darstellung die Auswertung für sechs Überwachungs-Zeiträume U_Zr.1, ..., U_Zr.6. Jede einzelne Darstellung ist so aufgebaut wie in Figur 8 gezeigt.

Figur 10 zeigt beispielhaft, wie die jeweilige Verfügbarkeitsrate Vr.1, ..., Vr.12 einer Abgasreinigungsanlage ARA in einem Überwachungs-Zeitraum dargestellt wird. Gezeigt werden zwölf Überwachungs-Zeiträume U_Zr.1, ..., U_Zr.12, die beispielsweise jeweils die Dauer von einem Monat haben. Das Auswertungsprogramm 8 berechnet die Verfügbarkeitsrate Vr.i der Abgasreinigungsanlage ARA im Überwachungs-Zeitraum U_Zr.i gemäß der Rechenvorschrift

$$(3) \quad Vr.i = 1 - \{dur[F\_Zr.1] + \ldots + dur[F\_Zr.k(i)]\} / dur(U\_Zr.i).$$

Hierbei sind F_Zr.1, ... F_Zr.k(i) die Fehler-Zeiträume, die im Überwachungs-Zeitraum U_Zr.i auftreten, dur[F_Zr] die zeitliche Dauer eines Fehler-Zeitraums F_Zr und dur(U_Zr.i) die zeitliche Dauer des Überwachungs-Zeitraums U_Zr.i.

Die Anzahl k(i) der Fehler-Zeiträume kann natürlich von Überwachungs-Zeitraum zu Überwachungs-Zeitraum variieren und auch 0 betragen.

In einer Ausgestaltung wird aus den letzten 12 Verfügbarkeitsraten Vr.1, ..., Vr.12 eine durchschnittliche Verfügbarkeitsrate Vr.avg für die letzten 12 Monate hergeleitet. Die durchschnittliche Verfügbarkeitsrate Vr.avg wird als gewichtetes Mittel berechnet, also gemäß der Rechenvorschrift

$$(4) \quad Vr.avg = \alpha.1 * Vr.1 + \ldots + \alpha.12 * Vr.12.$$

In einer Ausgestaltung werden die Gewichtsfaktoren $\alpha.1$, ..., $\alpha.12$ insbesondere anhand der Randbedingung ermittelt und vorgegeben, dass in einem landwirtschaftlichen Betrieb der Volumenstrom aus einem Stall heraus im Sommer in der Regel größer ist als im Winter. Möglich ist auch, dass alle Gewichtsfaktoren gleich groß sind, dass also ein arithmetisches Mittel gebildet wird.

Möglich ist, dass ein Rohgas-Sensor und / oder ein Reingas-Sensor fehlerhaft arbeiten oder sogar vollständig ausgefallen sind. In aller Regel liefert ein fehlerhafter Sensor einen zu niedrigen Ammoniak-Gehalt, ein ausgefallener Sensor sogar einen Ammoniak-Gehalt von 0, aber nicht einen zu hohen Ammoniak-Gehalt.

Das Auswertungsprogramm 8 auf der Recheneinheit 4 oder auch ein Auswertungsprogramm der Überwachungseinheit Ue.1, Ue.2 können in manchen Fällen automatisch einen Fehler und einen Ausfall eines Sensors erkennen und bis zum gewissen Grad kompensieren. Folgende Ereignisse sind ein Indiz für einen Fehler eines Sensors:

- Der oder ein Reingas-Sensor einer Überwachungseinheit Ue.1, Ue.2 liefert einen größeren Ammoniak-Gehalt als der oder ein Rohgas-Sensor dieser Überwachungseinheit Ue.1, Ue.2, und zwischen den beiden Abtastzeitpunkten dieser beiden Sensoren liegt eine Zeitspanne, die kleiner als eine vorgegebene obere Schranke ist. In der Regel ist dann der Rohgas-Sensor defekt oder fehlerhaft.
  Die Zeitspanne wird berücksichtigt, weil in einer größeren Zeitspanne der Ammoniak-Gehalt im Rohgas tatsächlich abnehmen kann.
- Der zeitlicher Verlauf der Ammoniak-Konzentration im Reingas und / oder im Rohgas sinkt schneller als eine vorgegebene Veränderungs-Schranke. Dies ist häufig ein Indiz dafür, das der Sensor, der diese Ammoniak-Konzentration gemessen hat, defekt geworden ist. Die Veränderungs-Schranke ist so gewählt, dass ein Absinken schneller als die Veränderungs-Schranke nicht mit dem tatsächlichen Verlauf einer Ammoniak-Konzentration übereinstimmen kann.
- Eine Überwachungseinheit Ue.1, Ue.2 umfasst zwei Rohgas-Sensoren. Zu einem Abtast-Zeitpunkt ist die absolute oder prozentuale Abweichung zwischen den beiden Messwerten, also zwischen den beiden gemessenen Ammoniak-Gehalten, dieser beiden Rohgas-Sensoren größer als eine vorgegebene untere Schranke. Oder die absolute oder prozentuale Abweichung wächst stärker als eine vorgegebene untere Schranke an.
  Dies ist ein Indiz dafür, dass derjenige Rohgas-Sensor, der den kleineren Ammoniak-Gehalt liefert, fehlerhaft arbeitet oder gar vollständig ausgefallen ist.
- Das Entsprechende gilt für eine Überwachungseinheit Ue.1, Ue.2 mit zwei Reingas-Sensoren.

Das Auswertungsprogramm 8 reagiert wie folgt auf die Detektion, dass ein Sensor fehlerhaft ist:

- Falls eine Überwachungseinheit Ue.1, Ue.2 nur einen einzigen Rohgas-Sensor aufweist und dieser ausgefallen ist, so generiert das Auswertungsprogramm 8 eine Fehlermeldung. Das entsprechende gilt, falls eine Überwachungseinheit Ue.1, Ue.2 nur einen einzigen Reingas-Sensor aufweist und dieser Sensor ausgefallen ist.
- Falls eine Überwachungseinheit Ue.1, Ue.2 zwei Rohgas-Sensoren aufweist und einer hiervon fehlerhaft misst oder ausgefallen ist, so verwendet das Auswertungsprogramm 8 den Messwert desjenigen Sensors, der einen größeren Ammoniak-Gehalt misst. Das Entsprechende gilt für eine Überwachungseinheit Ue.1, Ue.2 mit zwei Reingas-Sensoren. Denn ein Fehler oder ein Ausfall eines Sensors führt in der Regel zu einem zu geringen Messwert für den Ammoniak-Gehalt, aber nicht zu einem zu großen Messwert.

- Falls eine Überwachungseinheit Ue.1, Ue.2 zwei Rohgas-Sensoren umfasst und diese beiden Sensoren intakt sind, so ermittelt das Auswertungsprogramm 8 den Ammoniak-Gehalt im Rohgas durch eine Mittelung, z.B. eine gewichtete Mittelung, über die Messwerte dieser beiden Rohgas-Sensoren. Das Entsprechende gilt für eine Überwachungseinheit mit zwei Reingas-Sensoren.

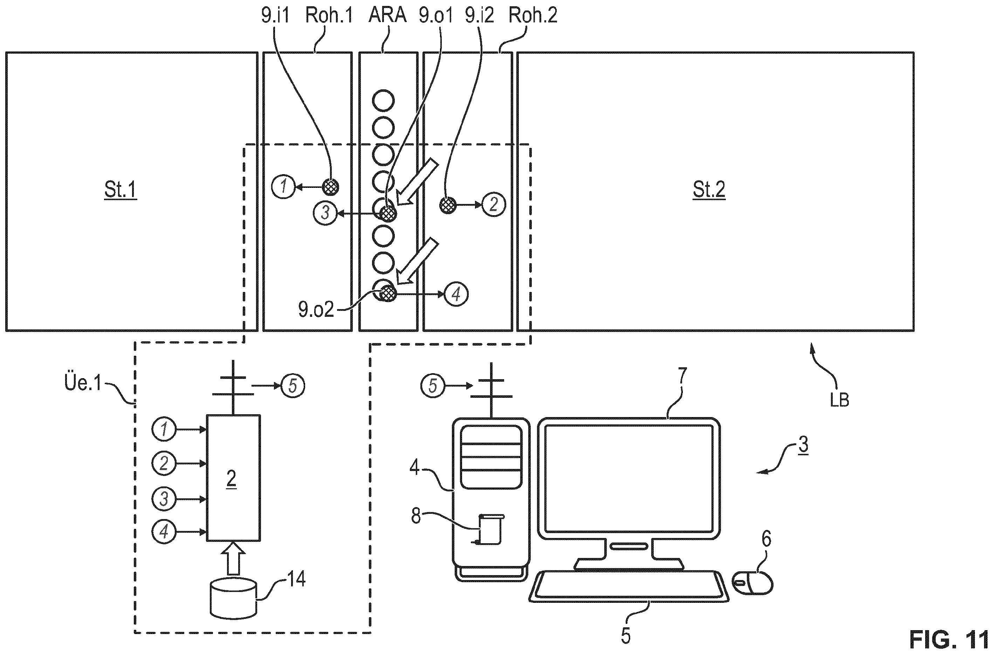

Nachfolgend wird mit Bezug auf Figur 11 eine Ausgestaltung beschrieben, bei der sich insbesondere die Datenverbindungen der erfindungsgemäßen Überwachungs-Anordnung überprüfen lassen. Die nachfolgende Beschreibung bezieht sich auf die Ausgestaltung gemäß Figur 1, bei der zwei Rohgas-Sensoren 1.i1, 1.i2 und zwei Reingas-Sensoren 1.o1, 1.o2 verwendet werden, um die Abgasreinigungsanlage ARA zu überprüfen. Gleiche Bezugszeichen haben die gleichen Bedeutungen wie in Figur 1. Die Überprüfung lässt sich entsprechend auch auf die Ausgestaltung gemäß Figur 2 verwenden.

Die Überwachungs-Anordnung lässt sich gemäß der nachfolgend beschriebenen Ausgestaltung wahlweise in einem Überwachungs-Modus oder in einem Überprüfungs-Modus betreiben. Im Überwachungs-Modus generiert und liefert jeder Ammoniak-Sensor 1.i1, 1.i2, 1.o1, 1.o2 jeweils ein Signal. Dieses Signal umfasst eine Information über den jeweils gemessenen Ammoniak-Gehalt. Das Auswertungsprogramm 8 ermittelt für die Abgasreinigungsanlage ARA und optional für jede weitere überwachte Abgasreinigungsanlage ARA' den jeweiligen Ammoniak-Gehalt im Rohgas und den im Reingas, und zwar für jeden Abtastzeitpunkt, so wie dies weiter oben beschrieben wurde.

Nachfolgend wird beschrieben, wie die Überwachungs-Anordnung im Überprüfungs-Modus betrieben wird. Im Überprüfungs-Modus wird jeder Ammoniak-Sensor 1.i1, 1.i2, 1.o1, 1.o2 durch jeweils einen Signalgeber 9.i1, 9.i2, 9.o1, 9.o2 ersetzt. Bevorzugt wird vorab sichergestellt, dass jeder Signalgeber 9.i1, 9.i2, 9.o1, 9.o2 fehlerfrei arbeitet. Nachfolgend werden die Begriffe Rohgas-Signalgeber 9.i1, 9.i2 und Reingas-Signalgeber 9.o1, 9.o2 verwendet.

In einer Ausgestaltung gehört jeder Ammoniak-Sensor 1.i1, 1.i2, 1.o1, 1.o2 physikalisch durch jeweils einen Signalgeber 9.i1, 9.i2, 9.o1, 9.o2 ersetzt. In einer anderen Ausgestaltung umfasst die erste Überwachungseinheit Ue.1 dauerhaft sowohl jeden Ammoniak-Sensor 1.i1, 1.i2, 1.o1, 1.o2 als auch jeden Signalgeber 9.i1, 9.i2, 9.o1, 9.o2. Im Überwachungs-Modus sind die Ammoniak-Sensoren 1.i1, 1.i2, 1.o1, 1.o2 aktiviert und die Signalgeber 9.i1, 9.i2, 9.o1, 9.o2 deaktiviert. Umgekehrt sind beim Betrieb im Überprüfungs-Modus die Ammoniak-Sensoren deaktiviert und die Signalgeber 9.i1, 9.i2, 9.o1, 9.o2 aktiviert. In einer Realisierungsform ist jeder Signalgeber 9.i1, 9.i2, 9.o1, 9.o2 ein Bestandteil jeweils eines Ammoniak-Sensors 1.i1, 1.i2, 1.o1, 1.o2, beispielsweise eine elektronische Schaltung.

Jeder Signalgeber 9.i1, 9.i2, 9.o1, 9.o2 umfasst bevorzugt eine eigene Spannungsversorgungseinheit und liefert ebenfalls jeweils ein Signal, welches eine Information über einen Ammoniak-Gehalt umfasst, wobei das Signal in einer Ausgestaltung ein elektrisches Signal ist und in einer anderen Ausgestaltung ein digitales Signal, welches das gleiche Datenformat aufweist wie das Signal eines Ammoniak-Sensors 1.i1, 1.i2, 1.o1, 1.o2.

Dieser Ammoniak-Gehalt im generierten Signal wurde aber nicht gemessen, sondern von einem Benutzer oder auch vom Auswertungsprogramm 8 oder von einer sonstigen Einheit vorgegeben. Das Signal, das der Signalgeber liefert, umfasst also eine Information über einen von einem Benutzer vorgegebenen Ammoniak-Gehalt. Bevorzugt umfasst jeder Signalgeber 9.i1, 9.i2, 9.o1, 9.o2 jeweils eine Eingabeeinheit, mit der ein Benutzer einen Ammoniak-Gehalt vorgeben kann. Möglich ist auch, dass sich jeder Signalgeber 9.i1, 9.i2, 9.o1, 9.o2 aus der Ferne ansteuern lässt, beispielsweise vom Auswertungsprogramm 8, und dem Signalgeber 9.i1, 9.i2, 9.o1, 9.o2 durch diese Ansteuerung ein Ammoniak-Gehalt vorgegeben werden kann. Jeder Signalgeber 9.i1, 9.i2, 9.o1, 9.o2 ersetzt und emuliert also jeweils einen Ammoniak-Sensor 1.i1, 1.i2, 1.o1, 1.o2.

Wie oben dargelegt wird, ermittelt das Auswertungsprogramm 8 beim Betrieb im Überwachungs-Modus für jeden Abtastzeitpunkt den jeweiligen Ammoniak-Gehalt im Rohgas und den im Reingas. Hierfür verwendet das Auswertungsprogramm 8 empfangene Signale der Ammoniak-Sensoren 1.i1, 1.i2, 1.o1, 1.o2. Außerdem berechnet das Auswertungsprogramm 8 für jeden Abtastzeitpunkt jeweils eine Gütefunktion in Form eines Reinigungsgrades $Rg = Rg(Amm_{rein}, Amm_{roh})$.

Im Überprüfungs-Modus wird dem oder jedem Rohgas-Signalgeber 9.i1, 9.i2 jeweils ein Ammoniak-Gehalt im Rohgas vorgegeben. Bei mehreren Rohgas-Signalgebern wird in einer Realisierungsform jedem Rohgas-Signalgeber 9.i1, 9.i2 der gleiche Ammoniak-Gehalt $Amm_{roh}$ im Rohgas vorgegeben, in einer anderen Realisierungsform zwei verschiedene Werte für den Ammoniak-Gehalt, beispielsweise $Amm_{roh} + \Delta$ bzw. $Amm_{roh} - \Delta$. Dadurch lässt sich zusätzlich überprüfen, ob das Auswertungsprogramm 8 gemessene Werte richtig zu einem einzigen Wert aggregiert. Das entsprechende gilt für die Reingas-Signalgeber 9.o1, 9.o2.

Die Signale mit den jeweils vorgegebenen Werten für den Ammoniak-Gehalt werden von den Signalgebern 9.i1, 9.i2, 9.o1, 9.o2 an den Zentralrechner 3 übermittelt und vom Auswertungsprogramm 8 ausgewertet. Auch beim Betrieb im Überprüfungs-Modus ermittelt das Auswertungsprogramm 8 jeweils einen Ammoniak-Gehalt. Das Auswertungsprogramm 8 verwendet für diese Ermittlung die empfangenen Signale der Signalgeber 9.i1, 9.i2, 9.o1, 9.o2.

Das Auswertungsprogramm 8 ermittelt einen Wert für den Ammoniak-Gehalt im Rohgas, wofür das Auswertungsprogramm 8 die Signale der Rohgas-Signalgeber 9.i1, 9.i2 verwendet. Entsprechend ermittelt das Auswertungsprogramm 8 einen Wert für den Ammoniak-Gehalt im Reingas, wofür das Auswertungsprogramm 8 die Signale der Reingas-Signalgeber 9.o1, 9.o2 verwendet. In einer Realisierungsform "weiß" das Auswertungsprogramm 8 für jeden Signalgeber 9.i1, 9.i2, 9.o1, 9.o2, welcher Wert für einen Ammoniak-Gehalt diesem Signalgeber vorgegeben worden ist. Das Auswertungsprogramm 8 vergleicht die ermittelten Werte für den Ammoniak-Gehalt, die durch Auswertung der Signale der Signalgeber 9.i1, 9.i2, 9.o1, 9.o2 ermittelt worden sind, mit den entsprechenden vorgegebenen Werten. In einer anderen Realisierungsform führt ein Benutzer diesen Vergleich durch.

Bei beiden Realisierungsformen wird geprüft, ob der ermittelte Wert für einen Ammoniak-Gehalt von dem entsprechenden vorgegebenen Wert um mehr als eine vorgegebene Toleranz abweicht. Mögliche Ursachen für eine Abweichung größer als eine vorgegebene Toleranz sind insbesondere:

- Ein Bestandteil der Überwachungs-Anordnung wird überhaupt nicht oder nicht ausreichend mit elektrischer Spannung versorgt. Dies gilt sowohl für einen Bestandteil, der eine eigene Spannungsversorgungseinheit aufweist, als auch für einen Bestandteil, der wenigstens zeitweise an ein stationäres Spannungsversorgungsnetz angeschlossen ist.
- Eine Datenverbindung von einem Signalgeber 9.i1, 9.i2, 9.o1, 9.o2 zum Zentralrechner 3 ist unterbrochen oder auf eine andere Weise defekt.
- Eine Signalverarbeitung auf dem Weg von einem Signalgeber 9.i1, 9.i2, 9.o1, 9.o2 zum Auswertungsprogramm 8 liefert fehlerhafte Werte.

In einer Fortbildung der gerade beschriebenen Ausgestaltung wird jedem Signalgeber 9.i1, 9.i2, 9.o1, 9.o2 jeweils ein zeitlicher Verlauf eines Ammoniak-Gehalts vorgegeben. Bevorzugt wird dadurch der jeweils vorgegebene Wert für den Ammoniak-Gehalt dergestalt variiert, dass er mindestens einmal den Wert null und mindestens einmal den größtmöglichen Wert für den Ammoniak-Gehalt annimmt. Dadurch wird insbesondere die Signalverarbeitung für den gesamten möglichen Wertebereich des Ammoniak-Gehalts überprüft, und zwar sowohl für das Rohgas als auch für das Reingas. Dadurch, dass zeitliche Verläufe verwendet werden, lässt sich auch besser eine erhebliche zeitliche Verzögerung bei der Datenübermittlung erkennen, als wenn nur jeweils ein einziger Wert vorgegeben werden würde.

Bevorzugt vermag das Auswertungsprogramm 8 automatisch zu erkennen, ob aktuell Ammoniak-Sensoren 1.i1, 1.i2, 1.o1, 1.o2 oder Signalgeber 9.i1, 9.i2, 9.o1, 9.o2 verwendet werden, ob also die Überwachungs-Anordnung aktuell im Überwachungs-Modus oder im Überprüfungs-Modus betrieben wird. Beispielsweise umfasst jedes Signal eines Signalgebers 9.i1, 9.i2, 9.o1, 9.o2 zusätzlich zum vorgegebenen Ammoniak-Gehalt jeweils eine entsprechende Information, beispielsweise eine Kennung des Signalgebers 9.i1, 9.i2, 9.o1, 9.o2 oder eine Information, dass der Ammoniak-Gehalt im übermittelten Signal vorgegeben und nicht gemessen worden ist. Möglich ist auch, jedem Signalgeber 9.i1, 9.i2, 9.o1, 9.o2 als zeitlichen Verlauf des Ammoniak-Gehalts ein Testmuster vorzugeben, welches in einem realen Betrieb der überwachten Abgasreinigungsanlage ARA nicht auftritt.

**Bezugszeichenliste**

| 1 | Ammoniak-Sensor |
|---|---|
| 1.i1, 1.i2 | Rohgas-Sensoren der ersten Überwachungseinheit Ue.1, flussaufwärts von der Abgasreinigungsanlage ARA angeordnet |
| 1'.i1, 1'.i2 | Rohgas-Sensoren der weiteren Überwachungseinheit Ue.2, flussaufwärts von der Abgasreinigungsanlage ARA' angeordnet |
| 1.o1, 1.02 | Reingas-Sensoren der ersten Überwachungseinheit Ue.1, flussabwärts von der Abgasreinigungsanlage ARA angeordnet |
| 1'.o1 | Reingas-Sensor der weiteren Überwachungseinheit Ue.2, flussabwärts von der Abgasreinigungsanlage ARA' angeordnet |
| 2 | Kommunikationseinheit, empfängt Signale von den Rohgas-Sensoren 1.i1, 1.i2 und von den Reingas-Sensoren 1.o1, 1.o2, erzeugt für jedes Signal jeweils eine eindeutige Sensor-Kennung und übermittelt die Signale mit den Sensor-Kennungen an den Zentralrechner 3 |
| 3 | Zentralrechner 3, umfasst die Recheneinheit 4, den Bildschirm 7, die Tastatur 5 und die Maus 6, empfängt Signale von der Kommunikationseinheit 2 |
| 4 | Recheneinheit des Zentralrechners 3 |

(fortgesetzt)

| 5 | Tastatur des Zentralrechners 3 |
|---|---|
| 6 | Maus des Zentralrechners 3 |
| 7 | Bildschirm des Zentralrechners 3 |
| 8 | Auswertungsprogramm auf den Zentralrechner 3, wertet die Signale von den Sensoren 1.i1, 1.i2, 1.o1, 1.o2 aus |
| 8.1 | weiteres Auswertungsprogramm |
| 9.i1, 9.i2 | Rohgas-Signalgeber, welche beim Betrieb im Überprüfungs-Modus die Rohgas-Sensoren 1.i1, 1.i2 ersetzen |
| 9.o1, 9.o2 | Reingas-Signalgeber, welche beim Betrieb im Überprüfungs-Modus die Reingas-Sensoren 1.o1, 1.o2 ersetzen |
| 10 | Zuführeinheit, umfasst die Eintrittsöffnung 22 und die Austrittsöffnung 21 |
| 11 | Öse am Gehäuse 24 |
| 12 | Kabel am Gehäuse 24, ermöglicht es, den Ammoniak-Sensor 1 elektrisch mit einem stationären Spannungsversorgungsnetz zu verbinden |
| 14 | Datenspeicher, in dem die eindeutigen Kennungen der Sensoren 1.i1, 1.i2, 1.o1, 1.o2 und die eindeutige Kennung der Überwachungseinheit Ue.1, Ue.2 abgespeichert sind und auf den die Kommunikationseinheit 2 wenigstens zeitweise Lesezugriff hat |
| 20 | Wandung der Zuführeinheit 10 |
| 21 | Austrittsöffnung aus der Zuführeinheit 10 |
| 22 | Eintrittsöffnung in die Zuführeinheit 10 |
| 23 | Bereich in der Zuführeinheit 10, in der die Gasprobe sich beruhigt |
| 24 | Gehäuse, umgibt den Innenbereich 140 mit der Spannungsversorgungseinheit, trägt die Öse 11 und das Halteseil 12 |
| 27 | Eintrittsspalt zwischen der Zuführeinheit 10 und dem Prallschutz 40 |
| 30 | Heizelement in der Wandung 20 |
| 40 | mechanischer Prallschutz unter der Zuführeinheit 10 |
| 50 | Messelektrode |
| 51 | Gegenelektrode |
| 52 | Referenzelektrode |
| 53 | Elektrolyt |
| 54 | Potentiostat |
| 55 | Anzeigeeinheit |
| 56 | poröse Membrane |
| 57 | Gehäuse |
| 100 | Sensorzelle mit der Messkammer, misst den Ammoniak-Gehalt |
| 110 | Bereich der Messkammer, der eine von unten zuströmenden Gasprobe aufnimmt, wird von der Wand 130, der Austrittsöffnung 21 und der Membrane 121 begrenzt, gehört zur Sensorzelle 100 |
| 120 | Schutzfilter vor der Sensorzelle 100 |
| 121 | Membrane, die den Bereich 110 für eine Gasprobe von einem Elektrolyten in der Messkammer trennt |
| 130 | Wand der Messkammer der Sensorzelle 100 |
| 140 | Innenbereich im Gehäuse 24, nimmt eine Spannungsversorgungseinheit und eine signalverarbeitende Auswerteinheit auf |

| | | |
|---|---|---|
| $Amm_{rein}$ | Ammoniak-Gehalt im Reingas, von den Reingas-Sensoren 1.o1, 1.o2 gemessen |
| $Amm_{roh}$ | Ammoniak-Gehalt im Rohgas, von den Rohgas-Sensoren 1.i1, 1.i2 gemessen |
| ARA, ARA' | Abgasreinigungsanlage, entfernt Ammoniak aus Rohgas und liefert dadurch Reingas, wird durch die Überwachungseinheit Ue.1, Ue.2 überwacht |
| Auf | Auffangbehälter, welche das Waschwasser mit dem Ammoniumsulfat aufnimmt, gehört zur Abgasreinigungsanlage ARA |
| Δ | Differenz aus dem Ammoniak-Gehalt $Amm_{roh}$ im Rohgas und im Ammoniak-Gehalt $Amm_{rein}$ im Reingas |
| dur(F_Zr) | zeitliche Dauer des Fehler-Zeitraums F_Zr |
| F_Zr, F_Zr1, ... | Fehler-Zeitraum, das ist ein Zeitraum, in dem der Reinigungsgrad Rg durchgehend kleiner ist als eine vorgegebene untere Schranke |
| Gb | Gebäude, welches den Stall St, den Technikraum Tr und die Abgasreinigungsanlage WW, Auf aufnimmt |
| Gg | Gasgemisch, in dem der Ammoniak-Gehalt gemessen wird |
| K | Anzahl der unterschiedlichen Wirkprinzipien einer Abgasreinigungsanlage, die das Auswertungsprogramm 8 auszuwerten vermag |
| LB, LB' | landwirtschaftlicher Betrieb mit der Abgasreinigungsanlage ARA, ARA' |
| LW | Leitfähigkeits-Sensor, der die elektrische Leitfähigkeit des Waschwassers im Auffangbehälter Auf misst |
| M | maximal mögliche Anzahl von Rohgas-Sensoren, die das Auswertungsprogramm 8 auszuwerten vermag |
| N | maximal mögliche Anzahl von Reingas-Sensoren, die das Auswertungsprogramm 8 auszuwerten vermag |
| pH | pH-Wert-Sensor, der den pH-Wert des Waschwassers im Auffangbehälter Auf misst |
| Rg | Reinigungsgrad der Abgasreinigungsanlage ARA |
| St, St.1, St.2 | Stall für die Tierzucht |
| Roh | Bereich neben der Abgasreinigungsanlage ARA', in dem sich Rohgas sammeln kann |
| Roh.1, Roh.2 | Bereich zwischen dem Stall St.1, St.2 und der Abgasreinigungsanlage ARA, in dem sich Rohgas sammeln kann |
| StE | Entlüftung für den Stall St, umfasst den ersten Ventilator Vent1 |
| StB | Belüftung für den Stall St |
| Tr | Technikraum |
| Ue.1 | erste Überwachungseinheit, umfasst die Rohgas-Sensoren 1.i1, 1.i2, die Reingas-Sensoren 1.o1, 1.o2 und die Kommunikationseinheit 2, überwacht die Abgasreinigungsanlage ARA |
| Ue.2 | zweite (weitere) Überwachungseinheit, umfasst die Rohgas-Sensoren 1'.i1, 1'.i2, den Reingas-Sensor 1'.o1 und die Kommunikationseinheit 2', überwacht die Abgasreinigungsanlage ARA' |
| U_Zr, U_Zr.1, ... | Überwachungs-Zeitraum |
| Vb | Verwirbelungsbereich, in dem der Rohgas-Sensor 1.i1 angeordnet ist |
| Vent1 | erster Ventilator, im Entlüftungsbereich StE angeordnet, saugt Rohgas aus dem Stall St ab |
| Vent2 | zweiter Ventilator, auf dem Dach des Gebäudes Gb angeordnet, saugt Reingas aus dem Gebäude Gb ab |
| Vr.i | Verfügbarkeitsrate der Abgasreinigungsanlage ARA im Überwachungszeitraum U_Zr.i |
| Vr.avg | durchschnittliche Verfügbarkeitsrate der Abgasreinigungsanlage ARA |

(fortgesetzt)

| WW | Waschwand der chemisch arbeitenden Abgasreinigungsanlage ARA, setzt dem Rohgas Schwefelsäure zu, gehört zur Abgasreinigungsanlage ARA |
|---|---|

**Patentansprüche**

1. Überwachungs-Anordnung zur Überwachung einer Abgasreinigungsanlage (ARA, ARA'),

   wobei die überwachte Abgasreinigungsanlage (ARA, ARA') dazu ausgestaltet ist,

   - in einem Gasgemisch, nachfolgend Rohgas genannt, welches Ammoniak enthält oder enthalten kann, den Ammoniak-Gehalt zu reduzieren und
   - dadurch ein Gasgemisch mit einem reduziertem Ammoniak-Gehalt, nachfolgend Reingas genannt, zu liefern,

   wobei die Überwachungs-Anordnung

   - eine erste Überwachungseinheit (Ue.1) und
   - eine signalverarbeitende Auswertungseinheit (8)

   umfasst,
   wobei die erste Überwachungseinheit (Ue.1)

   - mindestens einen Rohgas-Sensor (1.i1, 1.i2) und
   - mindestens einen Reingas-Sensor (1.o1, 1.o2)

   umfasst,
   wobei der oder jeder Rohgas-Sensor (1.i1, 1.i2) der ersten Überwachungseinheit (Ue.1) dazu ausgestaltet ist,

   - den Ammoniak-Gehalt im Rohgas zu messen und
   - ein Signal zu generieren,

   wobei das generierte Signal eine Information über den von diesem Sensor (1.i1, 1.i2) gemessenen Ammoniak-Gehalt im Rohgas umfasst,
   wobei der oder jeder Reingas-Sensor (1.o1, 1.o2) der ersten Überwachungseinheit (Ue.1) dazu ausgestaltet ist,

   - den Ammoniak-Gehalt im Reingas zu messen und
   - ein Signal zu generieren,

   wobei das generierte Signal eine Information über den von diesem Sensor (1.o1, 1.o2) gemessenen Ammoniak-Gehalt im Reingas umfasst,
   wobei die Überwachungs-Anordnung dazu ausgestaltet ist, die Signale der Rohgas-Sensoren (1.i1, 1.i2) und die Signale der Reingas-Sensoren (1.o1, 1.o2) der ersten Überwachungseinheit (Ue.1) an die Auswertungseinheit (8) zu übermitteln,
   wobei mindestens ein Überwachungs-Zeitraum (U_Zr), eine Gütefunktion und eine untere Schranke vorgegeben sind,
   wobei die Gütefunktion dergestalt vom Ammoniak-Gehalt im Reingas und vom Ammoniak-Gehalt im Rohgas abhängt, dass ein Funktionswert der Gütefunktion umso größer ist, je kleiner bei gleichbleibendem Ammoniak-Gehalt im Rohgas der Ammoniak-Gehalt im Reingas ist, und
   wobei bevorzugt der Funktionswert umso größer ist, je kleiner der Quotient aus dem Ammoniak-Gehalt im Reingas und dem Ammoniak-Gehalt im Rohgas ist,
   wobei die Auswertungseinheit (8) dazu ausgestaltet ist,

   - abhängig von empfangenen Signalen den Ammoniak-Gehalt im Rohgas und den Ammoniak-Gehalt im Reingas zu ermitteln und
   - abhängig vom ermittelten Ammoniak-Gehalt im Rohgas und vom ermittelten Ammoniak-Gehalt im Reingas jeden Fehler-Zeitraum (F_Zr.1, ...), der im Überwachungs-Zeitraum (U_Zr) auftritt, zu ermitteln oder
- festzustellen, dass es im Überwachungs-Zeitraum (U_Zr) keinen Fehler-Zeitraum gibt,

wobei ein Fehler-Zeitraum (F_Zr.1, ...) ein Zeitraum ist, in dem jeder ermittelte Funktionswert der vorgegebenen Gütefunktion kleiner ist als die vorgegebene untere Schranke.

2. Überwachungs-Anordnung nach Anspruch 1,
**dadurch gekennzeichnet, dass**

die erste Überwachungseinheit (Ue.1) zwei Rohgas-Sensoren (1.i1, 1.i2) umfasst und
die Auswertungseinheit (8) dazu ausgestaltet ist zu überprüfen, ob ein vorgegebenes Ausfallkriterium für einen Rohgas-Sensor (1.i1, 1.i2) der ersten Überwachungseinheit (Ue.1) erfüllt ist,
wobei das Ausfallkriterium erfüllt ist,

- wenn die absolute oder prozentuale Abweichung zwischen dem Ammoniak-Gehalt, den ein Rohgas-Sensor (1.i1) der ersten Überwachungseinheit (Ue.1) gemessen hat, und dem Ammoniak-Gehalt, den der oder ein anderer Rohgas-Sensor (1.i2) der ersten Überwachungseinheit (Ue.1) gemessen hat, und / oder
- wenn die Veränderung dieser Abweichung

größer ist als eine vorgegebene untere Schranke, und
wobei die Auswertungseinheit (8) weiterhin dazu ausgestaltet ist,

- dann, wenn das Ausfallkriterium nicht erfüllt ist, den Ammoniak-Gehalt im Rohgas abhängig von den beiden Signalen der beiden Rohgas-Sensoren (1.i1, 1.i2) zu ermitteln, und
- dann, wenn das Ausfallkriterium erfüllt ist, den Ammoniak-Gehalt im Rohgas abhängig nur vom Signal, das als Information den größeren Ammoniak-Gehalt im Rohgas umfasst, zu ermitteln.

3. Überwachungs-Anordnung nach einem der vorhergehenden Ansprüche,
**dadurch gekennzeichnet, dass**

die erste Überwachungseinheit (Ue.1) zwei Reingas-Sensoren (1.o1, 1.o2) umfasst und
die Auswertungseinheit (8) dazu ausgestaltet ist zu überprüfen, ob ein vorgegebenes Ausfallkriterium für einen Reingas-Sensor (1.o1, 1.o2) der ersten Überwachungseinheit (Ue.1) erfüllt ist,
wobei das Ausfallkriterium erfüllt ist,

- wenn die absolute oder prozentuale Abweichung zwischen dem Ammoniak-Gehalt, den ein Reingas-Sensor (1.o1) der ersten Überwachungseinheit (Ue.1) gemessen hat, und dem Ammoniak-Gehalt, den der oder ein anderer Reingas-Sensor (1.o2) der ersten Überwachungseinheit (Ue.1) gemessen hat, und / oder
- wenn die Veränderung dieser Abweichung

größer ist als eine vorgegebene untere Schranke, und
wobei die Auswertungseinheit (8) weiterhin dazu ausgestaltet ist,

- dann, wenn das Ausfallkriterium nicht erfüllt ist, den Ammoniak-Gehalt im Reingas abhängig von den beiden Signalen der beiden Reingas-Sensoren (1.o1, 1.o2) zu ermitteln, und
- dann, wenn das Ausfallkriterium erfüllt ist, den Ammoniak-Gehalt im Reingas abhängig nur vom Signal, das den größeren Ammoniak-Gehalt im Reingas umfasst, zu ermitteln.

4. Überwachungs-Anordnung nach einem der vorhergehenden Ansprüche,
**dadurch gekennzeichnet, dass**

die Auswertungseinheit (8) dazu ausgestaltet ist,

- für den vorgegebenen Überwachungs-Zeitraum (U_Zr) jeweils zu ermitteln, wie lange im Überwachungs-Zeitraum (U_Zr) für die Abgasreinigungsanlage (ARA) die Funktionswerte der vorgegebenen Gütefunktion kleiner als die vorgegebene untere Schranke gewesen sind, und
- abhängig von dieser Ermittlung eine Verfügbarkeitsrate (Vr.1, ..., Vr.12) der Abgasreinigungsanlage (ARA) im Überwachungs-Zeitraum (U_Zr.1, ..., Ü_Zr.12) zu berechnen,

wobei die Verfügbarkeitsrate (Vr.1, ..., Vr.12) angibt, welchen zeitlichen Anteil diejenigen Zeiträume, in denen die Funktionswerte der Gütefunktion größer oder gleich der unteren Schranke gewesen sind, am Überwachungs-Zeitraum (U_Zr.1, ..., Ü_Zr.12) insgesamt hatten.

5. Überwachungs-Anordnung nach Anspruch 4,
**dadurch gekennzeichnet, dass**

eine Abfolge von mindestens zwei sich nicht überlappenden Überwachungs-Zeiträumen (U_Zr.1, ..., Ü_Zr.12) vorgegeben ist und
die Auswertungseinheit (8) dazu ausgestaltet ist,

- eine graphische Darstellung zu erzeugen und
- zu bewirken, dass die erzeugte graphische Darstellung in mindestens einer von einem Menschen wahrnehmbaren Form ausgegeben wird,

wobei die erzeugte graphische Darstellung für jeden vorgegebenen Überwachungs-Zeitraum (U_Zr.1, ..., Ü_Zr.12) der Abfolge zeigt, welche Verfügbarkeitsrate (Vr.1, ..., Vr.12) die Abgasreinigungsanlage (ARA) in diesem Überwachungs-Zeitraum (U_Zr.1, ..., Ü_Zr.12) jeweils erzielt hat.

6. Überwachungs-Anordnung nach einem der vorhergehenden Ansprüche,
**dadurch gekennzeichnet, dass**

die Auswertungseinheit (8) dazu ausgestaltet ist,

- eine graphische Darstellung mit einer ersten Achse für die Zeit und einer zweiten Achse für den ermittelten Ammoniak-Gehalt zu erzeugen und
- zu bewirken, dass die erzeugte Darstellung in mindestens einer von einem Menschen wahrnehmbaren Form ausgegeben wird,

wobei die graphische Darstellung den zeitlichen Verlauf des ermittelten Ammoniak-Gehalts im Reingas und jeden ermittelten Fehler-Zeitraum (F_Zr.1, ...) zeigt, und
wobei für jeden Fehler-Zeitraum (F_Zr.1, ...) derjenige Abschnitt des zeitlichen Verlaufs, der in diesen Fehler-Zeitraum (F_Zr.1, ...) fällt, auf eine erste Weise dargestellt ist und
wobei jeder Abschnitt des zeitlichen Verlaufs, der nicht in einen Fehler-Zeitraum (F_Zr.1, ...) fällt, auf eine von der ersten Weise abweichenden zweiten Weise dargestellt ist.

7. Überwachungs-Anordnung nach einem der vorhergehenden Ansprüche,
**dadurch gekennzeichnet, dass**

mindestens ein Sensor (1.i1, 1.i2, 1.o1, 1.o2), bevorzugt jeder Sensor, der ersten Überwachungseinheit (Ue.1) jeweils eine Sensorzelle (100) mit

- einer Messkammer (120, 121, 130),
- einer röhrenförmigen Zuführeinheit (10) und
- einem Heizelement (30)

umfasst,
wobei bei einem Einsatz der Überwachungs-Anordnung die Zuführeinheit (10) senkrecht oder schräg unterhalb der Messkammer (120, 121, 130) angeordnet ist,
wobei das Heizelement (30) dazu ausgestaltet ist,

- das Innere der Zuführeinheit (10) zu erhitzen und
- dadurch zu bewirken, dass in der Zuführeinheit (10) eine Konvektionsströmung erzeugt wird,

wobei die erzeugte Konvektionsströmung eine Gasprobe aus der Umgebung durch die Zuführeinheit (10) hindurch senkrecht oder schräg nach oben in die Messkammer (120, 121, 130) fördert, und
wobei die Sensorzelle (100) dazu ausgestaltet ist, den Ammoniak-Gehalt in einer Gasprobe in der Messkammer (120, 121, 130) zu messen.

8. Überwachungs-Anordnung nach einem der vorhergehenden Ansprüche,
**dadurch gekennzeichnet, dass**

die erste Überwachungseinheit (Ue.1) für mindestens einen Sensor (1.o1, 1.o2) ein röhrenförmiges Schutzelement umfasst,
wobei sich das Schutzelement entlang einer Längsachse erstreckt,
wobei die Längsachse des Schutzelements bei einem Einsatz der Überwachungs-Anordnung vertikal oder schräg angeordnet ist und
wobei der Sensor (1.o1, 1.o2) im Inneren des Schutzelements angeordnet ist.

9. Überwachungs-Anordnung nach einem der vorhergehenden Ansprüche,
**dadurch gekennzeichnet, dass**

die erste Überwachungseinheit (Ue.1) zusätzlich eine erste Kommunikationseinheit (2) umfasst und
die Überwachungs-Anordnung einen Zentralrechner (3) umfasst, der räumlich von der ersten Überwachungseinheit (Ue.1) beabstandet angeordnet ist,
wobei wenigstens zeitweise jeweils eine Datenverbindung zwischen jedem Sensor (1.i1, 1.i2, 1.o1, 1.o2) der ersten Überwachungseinheit (Ue.1) und der ersten Kommunikationseinheit (2) hergestellt oder herstellbar ist,
wobei wenigstens zeitweise eine Datenverbindung zwischen der ersten Kommunikationseinheit (2) und dem Zentralrechner (3) hergestellt oder herstellbar ist,
wobei die erste Kommunikationseinheit (2) dazu ausgestaltet ist,

- das jeweilige Signal von jedem Sensor (1.i1, 1.i2, 1.o1, 1.o2) der ersten Überwachungseinheit (Ue.1) zu empfangen und
- für jeden Sensor (1.i1, 1.i2, 1.o1, 1.o2) jeweils eine Nachricht zu generieren und an den Zentralrechner (3) zu übermitteln,

wobei die für einen Sensor (1.i1, 1.i2, 1.o1, 1.o2) generierte Nachricht eine Information über den von diesem Sensor (1.i1, 1.i2, 1.o1, 1.o2) gemessenen Ammoniak-Gehalt umfasst, und
wobei die Auswertungseinheit (8) ein Bestandteil des Zentralrechners (3) ist und / oder auf dem Zentralrechner (3) ausführbar ist.

10. Überwachungs-Anordnung nach Anspruch 9,
**dadurch gekennzeichnet, dass**

die Überwachungs-Anordnung mindestens eine weitere Überwachungseinheit (Ue.2) umfasst,
wobei der Zentralrechner (3) räumlich von der weiteren Überwachungseinheit (Ue.2) beabstandet angeordnet ist,
wobei die weitere Überwachungseinheit (Ue.2)

- mindestens einen weiteren Rohgas-Sensor (1'.i1),
- mindestens einen weiteren Reingas-Sensor (1'.o1, 1'.o2) und
- eine weitere Kommunikationseinheit (2')

umfasst,
wobei wenigstens zeitweise eine Datenverbindung zwischen der weiteren Kommunikationseinheit (2') und den Zentralrechner (3) hergestellt oder herstellbar ist und
wobei die erste Überwachungseinheit (Ue.1) einer ersten Abgasreinigungsanlage (ARA) und die weitere Überwachungseinheit (Ue.2) einer weiteren Abgasreinigungsanlage (ARA') zugeordnet ist.

11. Überwachungs-Anordnung nach Anspruch 9 oder Anspruch 10,
**dadurch gekennzeichnet, dass**

die erste Kommunikationseinheit (2) einen Datenspeicher (14) umfasst,
wobei im Datenspeicher (14) eine Kennung für die erste Kommunikationseinheit (2) und für jeden Sensor (1.i1, 1.i2, 1.o1, 1.o2) der die ersten Überwachungseinheit (Ue.1) jeweils eine Kennung abgespeichert sind,
wobei die Kennung der ersten Kommunikationseinheit (2) diese Kommunikationseinheit (2) von der oder jeder anderen Kommunikationseinheit (2'), die über eine Datenverbindung mit den Zentralrechner (3) wenigstens zeitweise verbunden oder verbindbar ist, unterscheidet,
wobei die Kennung eines Sensors (1.i1, 1.i2, 1.o1, 1.o2) der ersten Überwachungseinheit (Ue.1) diesen Sensor von jedem anderen Sensor der ersten Überwachungseinheit (Ue.1) unterscheidet und
wobei die erste Kommunikationseinheit (2) dazu ausgestaltet ist, jede Nachricht, die ein von einem Sensor (1.i1, 1.i2, 1.o1, 1.o2) der ersten Überwachungseinheit (Ue.1) empfangenes Signal umfasst,
so zu generieren, dass die Nachricht zusätzlich die Kennung der ersten Überwachungseinheit (Ue.1) und die Kennung dieses Sensors (1.i1, 1.i2, 1.o1, 1.o2) umfasst.

12. Überwachungs-Anordnung nach Anspruch 11,
**dadurch gekennzeichnet, dass**

im Datenspeicher (14) zusätzlich eine Anzahl-Kennung dafür abgespeichert ist, wie viele Rohgas-Sensoren und wie viele Reingas-Sensoren die erste Überwachungseinheit (Ue.1) umfasst,
wobei die erste Kommunikationseinheit (2) dazu ausgestaltet ist, jede Nachricht, die ein von einem Sensor (1.i1, 1.i2, 1.o1, 1.o2) der ersten Überwachungseinheit (Ue.1) empfangenes Signal umfasst,
so zu generieren, dass die Nachricht zusätzlich die Anzahl-Kennung für die beiden Anzahlen umfasst.

13. Überwachungs-Anordnung nach einem der vorhergehenden Ansprüche,
**dadurch gekennzeichnet, dass**

die erste Überwachungseinheit einen Leitfähigkeits-Sensor (LW) und / oder einen pH-Wert-Sensor (pH) umfasst,
wobei der Leitfähigkeits-Sensor (LW) dazu ausgestaltet ist,

- die elektrische Leitfähigkeit einer Reinigungsflüssigkeit, die zum Reduzieren des Ammoniak-Gehalts im Rohgas verwendet wird, zu messen, und
- ein Signal umfassend die gemessene elektrische Leitfähigkeit zu generieren,

wobei der pH-Wert-Sensor (pH) dazu ausgestaltet ist,

- den pH-Wert der Reinigungsflüssigkeit zu messen und
- ein Signal umfassend den gemessenen pH-Wert zu generieren,

wobei die Auswertungseinheit (8) dazu ausgestaltet ist,

- zusätzlich jeden Zeitraum im Überwachungs-Zeitraum (U_Zr) zu ermitteln, in dem der gemessene pH-Wert kleiner als eine vorgegebene untere Schranke ist und / oder ob die gemessene elektrische Leitfähigkeit größer als eine vorgegebene obere Schranke ist, und
- einen solchen Zeitraum ebenfalls als einen Fehler-Zeitraum zu verwenden.

14. Überwachungs-Anordnung nach Anspruch 13,
**dadurch gekennzeichnet, dass**

die erste Kommunikationseinheit (2) einen Datenspeicher (14) umfasst,
wobei im Datenspeicher (14) eine Kennung dafür abgespeichert ist, ob die von der ersten Überwachungseinheit (Ue.1) überwachte Abgasreinigungsanlage (ARA) biologisch oder chemisch arbeitet, und
wobei die Auswertungseinheit (8) dazu ausgestaltet ist, nur dann die gemessene elektrische Leitfähigkeit und den gemessenen pH-Wert mit der jeweiligen Schranke zu vergleichen, wenn die überwachte Abgasreinigungsanlage (ARA) biologisch arbeitet.

15. Überwachungs-Anordnung nach einem der vorhergehenden Ansprüche,
**dadurch gekennzeichnet, dass**

die Überwachungs-Anordnung für den oder jeden Rohgas-Sensor (1.i1, 1.i2) und für den oder jeden Reingas-Sensor (1.o1, 1.o2) der ersten Überwachungseinheit (Ue.1) jeweils einen Signalgeber (9.i1, 9.i2, 9.o1, 9.o2) umfasst,
wobei jeder Signalgeber (9.i1, 9.i2, 9.o1, 9.o2) dazu ausgestaltet ist,

- jeweils einen vorgegebenen Wert für einen Ammoniak-Gehalt zu erfassen und

- ein Signal dergestalt zu generieren, dass das generierte Signal eine Information über den diesem Signalgeber (9.i1, 9.i2, 9.o1, 9.o2) vorgegebenen Ammoniak-Gehalt umfasst,

wobei die Überwachungs-Anordnung wahlweise in einem Überwachungs-Modus oder in einem Überprüfungs-Modus betreibbar ist,
wobei die Überwachungs-Anordnung dazu ausgestaltet ist, beim Betrieb im Überwachungs-Modus den oder jeden Rohgas-Sensor (1.i1, 1.i2) und den oder jeden Reingas-Sensor (1.o1, 1.o2) der ersten Überwachungseinheit (Ue.1) zu verwenden, und
wobei die Überwachungs-Anordnung dazu ausgestaltet ist, beim Betrieb im Überprüfungs-Modus

- anstelle des oder jedes Rohgas-Sensors (1.i1, 1.i2) und des oder jedes Reingas-Sensors (1.o1, 1.o2) den jeweils zugeordneten Signalgeber (9.i1, 9.i2, 9.o1, 9.o2) zu verwenden und
- die Signale der Signalgeber (9.i1, 9.i2, 9.o1, 9.o2) an die Auswertungseinheit (8) zu übermitteln und

die Auswertungseinheit (8) beim Betrieb im Überprüfungs-Modus dazu ausgestaltet ist, abhängig von empfangenen Signalen jeden Wert für einen Ammoniak-Gehalt, der einem Signalgeber (9.i1, 9.i2, 9.o1, 9.o2) vorgegeben ist, zu ermitteln.

16. System umfassend

- mindestens eine Abgasreinigungsanlage (ARA, ARA') und
- eine Überwachungs-Anordnung nach einem der vorherigen Ansprüche,
wobei die oder jede Abgasreinigungsanlage (ARA, ARA') dazu ausgestaltet ist,

- in einem Gasgemisch welches Ammoniak enthält oder enthalten kann, den Ammoniak-Gehalt zu reduzieren und
- dadurch ein Gasgemisch mit einem reduziertem Ammoniak-Gehalt zu liefern, und

wobei mindestens einer, bevorzugt jeder Abgasreinigungsanlage (ARA, ARA') des Systems jeweils eine Überwachungseinheit (Ue.1, Üe.2) der Überwachungs-Anordnung zugeordnet ist und
wobei die Überwachungs-Anordnung dazu ausgestaltet ist, die oder jede Abgasreinigungsanlage (ARA, ARA') des Systems, der eine Überwachungseinheit (Ue.1, Üe.2) zugeordnet ist, zu überwachen.

17. System nach Anspruch 16,
**dadurch gekennzeichnet, dass**

das System ein Gebäude (Gb) umfasst,
wobei die oder mindestens eine überwachte Abgasreinigungsanlage (ARA) des Systems im Gebäude (Gb) oder angrenzend an das Gebäude angeordnet ist und
wobei im Gebäude (Gb) weiterhin ein Stall (St) für die Haltung von Tieren untergebracht ist.

18. Überwachungs-Verfahren zur Überwachung einer Abgasreinigungsanlage (ARA),

wobei die überwachte Abgasreinigungsanlage (ARA) dazu ausgestaltet ist,

- in einem Gasgemisch, nachfolgend Rohgas genannt, welches Ammoniak enthält oder enthalten kann, den Ammoniak-Gehalt zu reduzieren und
- dadurch ein Gasgemisch mit einem reduziertem Ammoniak-Gehalt, nachfolgend Reingas genannt, zu liefern,

wobei das Überwachungs-Verfahren unter Verwendung einer Überwachungs-Anordnung durchgeführt wird,
wobei die Überwachungs-Anordnung

- eine erste Überwachungseinheit (Ue.1) und
- eine signalverarbeitende Auswertungseinheit (8)

umfasst,
wobei die erste Überwachungseinheit (Ue.1)

- mindestens einen Rohgas-Sensor (1.i1, 1.i2) und
- mindestens einen Reingas-Sensor (1.o1, 1.o2)

umfasst,

wobei mindestens ein Überwachungs-Zeitraum (U_Zr), eine Gütefunktion und eine untere Schranke vorgegeben werden,

wobei die Gütefunktion dergestalt vom Ammoniak-Gehalt im Reingas und vom Ammoniak-Gehalt im Rohgas abhängt, dass der Funktionswert der Gütefunktion umso größer ist, je kleiner bei gleichbleibendem Ammoniak-Gehalt im Rohgas der Ammoniak-Gehalt im Reingas ist, und

wobei bevorzugt der Funktionswert umso größer ist, je kleiner der Quotient aus dem Ammoniak-Gehalt im Reingas und dem Ammoniak-Gehalt im Rohgas ist,

wobei das Verfahren die automatisch durchgeführten Schritte umfasst, dass

- der oder jeder Rohgas-Sensor (1.i1, 1.i2) der ersten Überwachungseinheit (Ue.1) wiederholt den Ammoniak-Gehalt im Rohgas misst und ein Signal generiert,
wobei das generierte Signal eine Information über den von diesem Rohgas-Sensor (1.i1, 1.i2) gemessenen Ammoniak-Gehalt im Rohgas umfasst,
- der oder jeder Reingas-Sensor (1.o1, 1.o2) der ersten Überwachungseinheit (Ue.1) wiederholt den Ammoniak-Gehalt im Reingas misst und ein Signal generiert,
wobei das generierte Signal eine Information über den von diesem Reingas-Sensor (1.o1, 1.o2) gemessenen Ammoniak-Gehalt im Reingas umfasst,
- die generierten Signale an die Auswertungseinheit (8) übermittelt werden,
- die Auswertungseinheit (8) abhängig von empfangenen Signalen den Ammoniak-Gehalt im Rohgas und den Ammoniak-Gehalt im Reingas ermittelt und
- die Auswertungseinheit (8) abhängig von den beiden ermittelten Ammoniak-Gehalten jeden Fehler-Zeitraum (F_Zr.1, ...), der im Überwachungs-Zeitraum (U_Zr) auftritt, ermittelt oder feststellt, dass es im Überwachungs-Zeitraum (U_Zr) keinen Fehler-Zeitraum gibt,

wobei ein Fehler-Zeitraum (F_Zr.1, ...) ein Zeitraum ist, in dem jeder ermittelte Funktionswert der vorgegebenen Gütefunktion kleiner ist als die vorgegebene untere Schranke.

19. Überwachungs-Verfahren nach Anspruch 18,
**dadurch gekennzeichnet, dass**

die erste Überwachungseinheit (Ue.1) zusätzlich eine erste Kommunikationseinheit (2) umfasst und die Überwachungs-Anordnung einen Zentralrechner (3) umfasst, der räumlich von der ersten Überwachungseinheit (Ue.1) beabstandet angeordnet ist,

wobei die Auswertungseinheit (8) ein Bestandteil des Zentralrechners (3) ist und / oder auf dem Zentralrechner (3) ausgeführt wird und

wobei das Verfahren die zusätzlichen Schritte umfasst, dass

- wenigstens zeitweise jeweils eine Datenverbindung zwischen jedem Sensor (1.i1, 1.i2, 1.o1, 1.o2) der ersten Überwachungseinheit (Ue.1) und der ersten Kommunikationseinheit (2) hergestellt wird,
- wenigstens zeitweise eine Datenverbindung zwischen der ersten Kommunikationseinheit (2) und dem Zentralrechner (3) hergestellt wird und
- die erste Kommunikationseinheit (2) das jeweilige Signal von jedem Sensor (1.i1, 1.i2, 1.o1, 1.o2) der ersten Überwachungseinheit (Ue.1) empfängt und jeweils eine Nachricht umfassend von diesem Sensor gemessenen Ammoniak-Gehalt generiert und an den Zentralrechner (3) übermittelt,

wobei die Nachricht eine Information über den von diesem Sensor (1.i1, 1.i2, 1.o1, 1.o2) gemessenen Ammoniak-Gehalt umfasst.

20. Überwachungs-Verfahren nach Anspruch 18 oder Anspruch 19,
**dadurch gekennzeichnet, dass**

die verwendete Überwachungs-Anordnung für den oder jeden Rohgas-Sensor (1.i1, 1.i2) und für den oder jeden Reingas-Sensor (1.o1, 1.o2) der ersten Überwachungseinheit (Ue.1) jeweils einen Signalgeber (9.i1, 9.i2, 9.o1, 9.o2) umfasst,

wobei die Überwachungs-Anordnung mindestens einmal durch ein Überprüfungs-Verfahren überprüft wird, wobei das Überprüfungs-Verfahren die Schritte umfasst, dass

- anstelle des oder jedes Rohgas-Sensors (1.i1, 1.i2) und des oder jedes Reingas-Sensors (1.o1, 1.o2) der jeweils zugeordnete Signalgeber (9.i1, 9.i2, 9.o1, 9.o2) verwendet wird,
- jeder Signalgeber (9.i1, 9.i2, 9.o1, 9.o2) jeweils einen vorgegebenen Wert für einen Ammoniak-Gehalt erfasst und ein Signal generiert,
wobei das generierte Signal eine Information über den diesem Signalgeber (9.i1, 9.i2, 9.o1, 9.o2) vorgegebenen Ammoniak-Gehalt umfasst, und
- die von den Signalgebern (9.i1, 9.i2, 9.o1, 9.o2) generierten Signale an die Auswertungseinheit (8) übermittelt werden und
- die Auswertungseinheit (8) abhängig von empfangenen Signalen jeden Wert für einen Ammoniak-Gehalt, der einem Signalgeber (9.i1, 9.i2, 9.o1, 9.o2) vorgegeben ist, ermittelt.

**FIG. 1**

EP 4 653 673 A1

FIG. 2

EP 4 653 673 A1

FIG. 3

FIG. 4

FIG. 5

**FIG. 6**

1.o1

*Reingas*

Vent2

*Rohgas*

*Rohgas*

WW

Gb

Vent1

StE

StB

LB

Auf

ARA

Vb

1.i1

Tr

Lw

pH

St

EP 4 653 673 A1

FIG. 7

FIG. 8

FIG. 9

FIG. 10

EP 4 653 673 A1

FIG. 11

Europäisches
Patentamt

European
Patent Office

Office européen
des brevets

## EUROPÄISCHER RECHERCHENBERICHT

**Nummer der Anmeldung**

EP 25 17 7199

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 2 439 386 A1 (ZAYAN NICHOLAS MICHAEL [US]) 11. April 2012 (2012-04-11) * Anspruch 1; Abbildung 1 * ----- | 1-20 | INV. F01N11/00 G01N33/00 |
| A | CN 115 290 833 A (AGRO ENVIRONMENT PROT INSTITUTION MINISTRY OF AGRICULTURE AND RURAL AF) 4. November 2022 (2022-11-04) * Ansprüche 1-2; Abbildung 1 * ----- | 1-20 | |
| A | DE 10 2012 220152 A1 (BOSCH GMBH ROBERT [DE]) 22. Mai 2014 (2014-05-22) * Anspruch 1; Abbildung 1 * ----- | 1-20 | |
| A | EP 2 860 524 A1 (EXTOX GASMESS SYSTEME GMBH [DE]) 15. April 2015 (2015-04-15) * Anspruch 1; Abbildung 1 * ----- | 1-20 | |

**RECHERCHIERTE SACHGEBIETE (IPC)**

F01N
G01N

Der vorliegende Recherchenbericht wurde für alle Patentansprüche erstellt

| Recherchenort | Abschlußdatum der Recherche | Prüfer |
|---|---|---|
| München | 18. September 2025 | Seifert, Marco |

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 25 17 7199

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

18-09-2025

| Im Recherchenbericht angeführtes Patentdokument | Datum der Veröffentlichung | Mitglied(er) der Patentfamilie | Datum der Veröffentlichung |
|---|---|---|---|
| EP 2439386 A1 | 11-04-2012 | EP 2439386 A1<br>US 2012085083 A1 | 11-04-2012<br>12-04-2012 |
| CN 115290833 A | 04-11-2022 | KEINE | |
| DE 102012220152 A1 | 22-05-2014 | KEINE | |
| EP 2860524 A1 | 15-04-2015 | DE 102014005825 A1<br>EP 2860524 A1 | 09-04-2015<br>15-04-2015 |

EPO FORM P0461

Für nähere Einzelheiten zu diesem Anhang : siehe Amtsblatt des Europäischen Patentamts, Nr.12/82